(19) Europäisches Patentamt
European Patent Office
Office européen des brevets

(11) **EP 3 363 912 B1**

(12) **EUROPEAN PATENT SPECIFICATION**

(45) Date of publication and mention
of the grant of the patent:
**28.10.2020 Bulletin 2020/44**

(51) Int Cl.:
***C12Q 1/56*** *(2006.01)*     ***G01N 33/86*** *(2006.01)*

(21) Application number: **17156656.5**

(22) Date of filing: **17.02.2017**

(54) **SELF-CALIBRATED ASSAY FOR DETERMINING PROTEOLYTIC ENZYME GENERATION, DEDICATED MEANS AND USES THEREOF**

SELBSTKALIBRIERTER ASSAY ZUR BESTIMMUNG DER ERZEUGUNG VON PROTEOLYTISCHEM ENZYM, DEDIZIERTE MITTEL UND VERWENDUNGEN DAVON

DOSAGE À ÉTALONNAGE AUTOMATIQUE POUR DÉTERMINER LA PRODUCTION D'UNE ENZYME PROTÉOLYTIQUE, MOYENS DÉDIÉS ET UTILISATIONS ASSOCIÉES

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(43) Date of publication of application:
**22.08.2018 Bulletin 2018/34**

(73) Proprietor: **Synapse B.V.**
**6229 ER Maastricht (NL)**

(72) Inventor: **HEMKER, Hendrik Coenraad**
**6211 LM Maastricht (NL)**

(74) Representative: **Nederlandsch Octrooibureau**
**P.O. Box 29720**
**2502 LS The Hague (NL)**

(56) References cited:
**EP-A1- 2 677 037          WO-A1-00/52199**
**WO-A1-03/093831      WO-A2-2005/095638**
**US-A1- 2013 023 002**

• **M. NINIVAGGI ET AL: "Whole-Blood Thrombin Generation Monitored with a Calibrated Automated Thrombogram-Based Assay", CLINICAL CHEMISTRY, vol. 58, no. 8, 4 June 2012 (2012-06-04), pages 1252-1259, XP055038472, ISSN: 0009-9147, DOI: 10.1373/clinchem.2012.184077**

Printed by Jouve, 75001 PARIS (FR)

**Description**

**Field of the invention**

The invention pertains to the field of *in vitro* methods for investigating haemostasis and fibrinolysis, in particular for studying the processes involved in haemostatic and fibrinolytic systems.

**Background of the invention**

A class of important physiological processes (blood coagulation, complement activation, angiotensin development, fibrinolysis, intestinal digestion) consists of systems of proteolytic enzymes that are sequentially activated and subsequently inactivated. Such processes can be made to occur in samples of body fluids *in vitro* and the function of the physiological process can then be investigated by measuring the course of the activity of one or more of the enzymes as they appear and disappear in the course of time. This can be done by taking subsamples from the reaction medium and determining the enzyme in each of these subsamples consecutively taken at distinct times during evolution of the reaction medium. Alternatively, one can add a substrate that is split by the enzyme under study and that releases a product that can be quantitatively detected in real-time, e.g. via its fluorescence, optical density or electrochemical properties. From the velocity of product formation, the concentration of the enzyme then can be calculated. The principle is applicable to all proteolytic enzyme systems but has been elaborated in blood and plasma with regard to the haemostatic system, which depends on the generation of the enzyme thrombin and the fibrinolytic system, which dissolves clots and which depends on the enzyme plasmin.

Physiologically important enzymes such as thrombin (responsible for haemostasis and thrombosis) or plasmin (responsible for the removal of fibrin clots and thrombi) can be made to appear in a sample of blood or plasma or other body fluids by methods known to the art. In the course of time such enzymes disappear again by the action of natural inhibiting proteins that occur is such samples. The time course of enzyme activity, the so called thrombin- or plasmin-generation curve gives important insights in the function of the haemostatic- thrombotic- and fibrinolytic function in the sample and consequently in the subject (patient) that the sample is taken from.

*THROMBIN*

It is well known that thrombin plays a pivotal role in haemostasis and thrombosis. The mechanism of thrombin formation is extremely complex and replete with positive and negative feedback reactions.

For assessing the function of the haemostatic system, thrombin generation (TG) can be investigated in a form rendering the main parameters used for investigating the haemostatic system available altogether to the experimenter. For this purpose, use is conveniently made of curves representing the activity of thrombin against time (activity over time curve), also known under the name Thrombogram™ in the literature.

The result of the whole process of thrombin generation is indeed the appearance and disappearance of a transient thrombin activity. Thrombin generation results from the steps of thrombin appearance and disappearance of thrombin reflecting transient thrombin activity. This process of thrombin generation can occur in a blood sample *in vitro* when appropriate conditions enable the reactions of the coagulation cascade to take place.

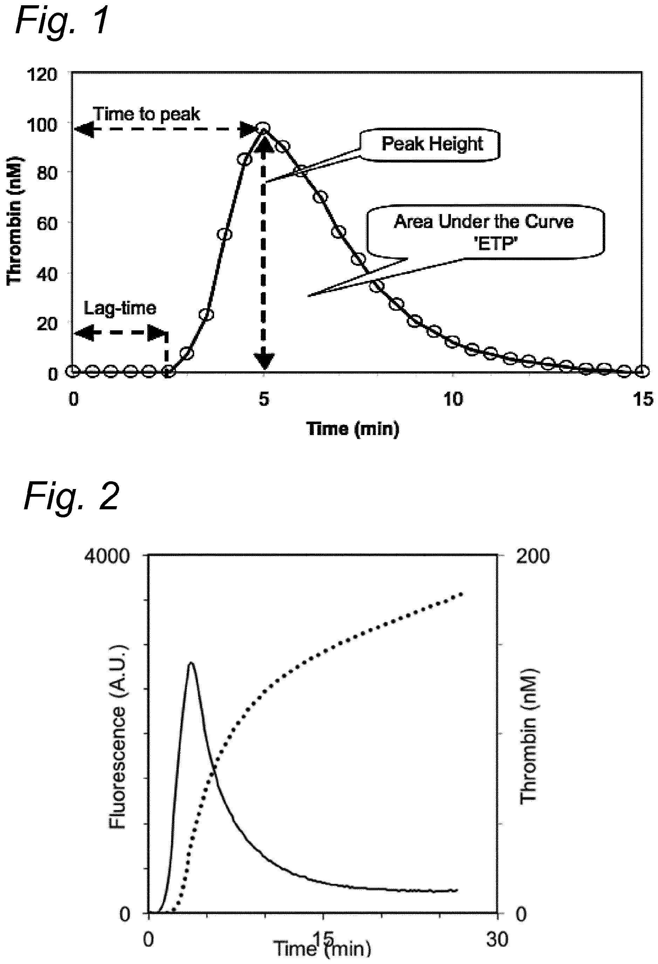

A typical curve of thrombin activity against time conveniently exploitable for interpretation by one skilled in the art, also called "thrombin generation curve", such as represented in Figs 1 and 2, is characterised by an initiation phase, or lag-time, during which only minute amounts of thrombin are formed; then follows a burst of activity, known as the propagation phase. Blood clots at the very beginning of the burst; almost all thrombin is formed after the clot has formed. All formed thrombin is subsequently inactivated by the antithrombins of the blood. These proteins bind stoichiometrically to thrombin in a reaction that is slow compared to thrombin formation. The inactivation velocity is proportional to the concentration of thrombin and of antithrombins. As long as the conversion rate of prothrombin is higher than the inactivation rate of thrombin the level of thrombin increases. As the level of thrombin increases the inactivation rate also increases. At the maximal thrombin concentration both velocities are equal; thereafter thrombin decay predominates. A typical curve of thrombin activity directly exploitable for interpretation by one skilled in the art therefore generally shows the above-mentioned several phases and especially shows the lag time before thrombin generation starts, the peak of thrombin generation, the time to reach the peak and the ETP (Endogenous Thrombin Potential), i.e. the area under the curve.

*PLASMIN*

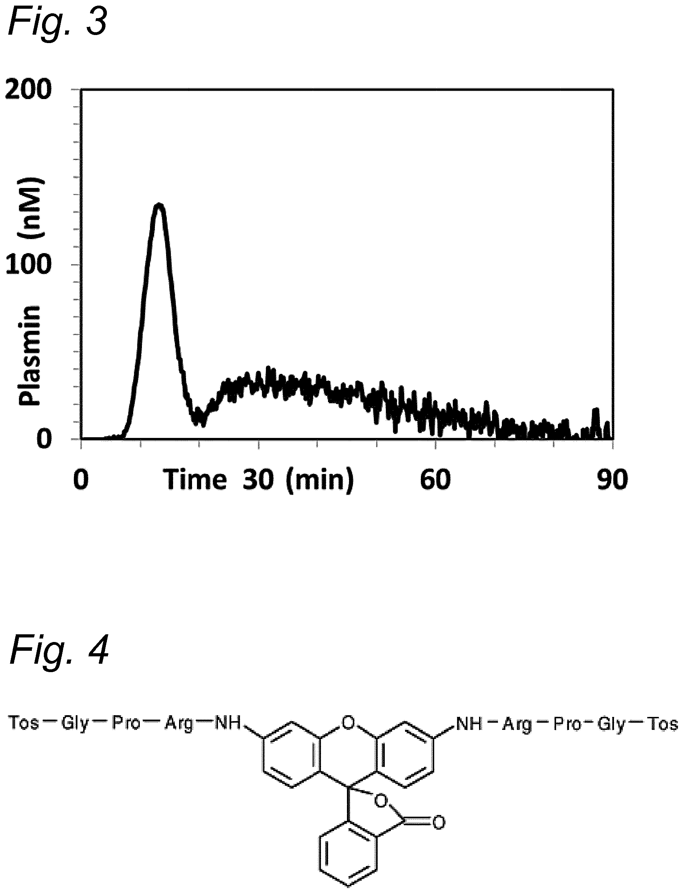

Similarly, curves of plasmin activity against time are known and conveniently used in the art. A typical plasmin generation curve such as represented in Fig. 3 is obtained when a fluorogenic substrate that is specific for plasmin and not for thrombin is added to plasma and coagulation is triggered as in a thrombin generation curve but also tissue plasminogen activator (tPA) is added. Plasmin generation is dependent upon the presence of tPA and fibrin, so that it starts after the sample has clotted. In contrast to the thrombin generation curve, the plasmin generation curve is often biphasic. The resulting graph is best characterised by its lag time, the height and position of its peaks and the areas under the peaks.

## State of the art and drawbacks of the state of the art

Establishing the time course of thrombin in clotting blood or plasma was done from the 1950ies on by measuring thrombin in samples taken from the clotting blood or plasma. This is a labor- and time demanding procedure that is unsuitable for large-scale applications such as patient diagnostics and pharmaceutical research.

Hemker and Béguin launched the principle of continuously monitoring the development of thrombin in a sample of triggered plasma in 1993 (Hemker HC, Wielders S, Kessels H. Béguin S: Continuous registration of thrombin generation in plasma, its use for the determination of the thrombin potential, Thromb.Haemost 1993, 70:617-624). They used thrombin substrates that released a colored product (p-nitroaniline) to be monitored via its optical density. This had the obvious disadvantage that the turbidity caused by the forming clot disturbs the measurement. The experiment could therefore only be carried out in plasma from which fibrinogen had been previously removed. This also removes cells, notably blood platelets that play an important role in thrombin generation because they provide the phospholipid surface on which thrombin generation reactions take place and have to be replaced by added phospholipids.

The same investigators circumvented this disadvantage when they replaced the chromogenic substrates by fluorogenic substrates, as these were not hindered by fibrin turbidity and thus allowed measurement in plasma containing fibrinogen and blood platelets (Hemker HC, Giesen PL, Ramjee M, Wagenvoord R, Béguin S: The thrombogram, monitoring thrombin generation in platelet-rich plasma, Thromb.Haemost 2000, 83:589-591).

Adding a thrombin substrate and monitoring the release of product in time invariably results in a trace like shown in Fig. 2 (dotted line). The more thrombin is present the faster the signal increases. In the end a residual activity remains because part of the thrombin is bound to $\alpha_2$-macroglobulin and remains capable to split the substrate.

The main problem with thrombin- (or plasmin) generation measurement(s) through added fluorogenic substrate(s) lies in the determination of the exact concentration of thrombin (plasmin) that causes the observed increase of fluorescence intensity. Optical Density (OD) changes can be directly related to concentration changes by methods known to the art but fluorescence changes hitherto required comparison to a standard. Moreover, the rate of change of fluorescence per unit thrombin decreases during the course of an experiment due to substrate consumption and to the non-linearity of fluorescence with product concentration (the inner filter effect). This makes that at every level of fluorescence a different relation exists between observed reaction velocity and enzyme (thrombin) concentration. This problem was solved in the technique disclosed in Hemker et al. (Thromb Haemost 2000; 83: 589-91) or Hemker et al. (Pathophysiol Haemost Thromb, 2002; 32: 249-253) or Hemker et al. (Pathophysiol Haemost Thromb, 2003; 33: 4-15) or International Patent Application WO 03/093831, and referred to as "Calibrated Automated Thrombinography" (CAT). In this technique the signal from the thrombin generation experiment is continuously compared to that of a parallel experiment in which a known and constant amount of thrombin-like activity (usually in the form of $\alpha$2macroglobulin-thrombin complex) is present. This has the obvious drawback of requiring two experiments running in parallel instead of one. Also, drugs that inhibit both thrombin and the $\alpha$2macroglobulin-thrombin complex, such as reversible small molecular weight thrombin inhibitors (e.g. Dabigatran) cannot be correctly quantified in this manner. How to derive the thrombin concentration from a comparison of the calibrator curve and the curve from the generation experiment is a problem in itself, which can be solved by mathematical means (International Patent Application WO 2009/098313).

A need therefore remains for a real-time assay avoiding the necessity to rely on a separate calibration experiment necessarily performed on a distinct, separated sample, i.e. a calibration experiment not performed on the assayed sample, that may also be considered as an "external" calibration experiment, distinct from the assayed sample, which is the sample on which investigation of thrombin and/or plasmin generation parameters is sought.

In this context, a need also remained for an assay that would enable investigating samples containing reversible thrombin inhibitors. Such inhibitors affect calibrator and experiment alike and therefore their effect cannot be deduced from a comparison of the two.

International Patent Application WO 2006/117246 discloses a real-time method for determining thrombin activity in a sample by assaying a fluorogenic substrate of thrombin in a layer of sample having a specific thickness and surface in order to deal with the inner filter effect, without, however, obviating the necessity of external calibration. WO 2006/117246 also discloses the addition of a known small amount of fluorescent product to the reaction mixture, so that it can serve to estimate the optically accessible volume from which fluorescence is registered. A later patent application also discloses the same type of use of the addition of product to the reaction mixture (WO 2007/141023). Neither of the two patent applications, nor any other prior art mentions the interest or possibility to make use of the native fluorescence of the substrate for purposes of internal calibration, thus avoiding external calibration. Nor is mentioned the possibility of calculating the amount of active enzyme directly from the ratio of the developing fluorescence to the fluorescence at zero-time. In fact, substrates with native fluorescence were not available and proved to have particular advantages, as exploited within the present invention, only when suitably used. The advantage being that the quantification of the enzyme does not rely on an amount of added product, which is subject to experimental variation, but to a fixed natural constant, i.e. the ratio of the fluorescence yield of the product over that of the substrate. Notably, the prior art does not provide a method that abolishes the need to carry out two parallel experiments. Neither does it provide a solution for measuring the effect of direct reversible thrombin inhibitors.

WO 2006/072602 teaches an assay for simultaneously determining two or more transiently occurring enzymes, in particular thrombin generation and plasmin generation, which may accordingly be simultaneously detected in one single well without, however, obviating the necessity for the measurement of one, or even two parallel samples for calibration purposes; two because in that method two different substrates are used simultaneously, one substrate specific for each enzyme.

In order to minimize quenching of fluorogenic substrate(s) by hemoglobin, so as to allow measurement of TG in whole blood, it is also known in the art to use Rhodamin 110 (Rho) as a fluorophore. This fluorophore has two identical reactive NH-groups that may each be substituted with arginine or lysine containing oligopeptides (Fig. 4), so as to make specific thrombin- or plasmin substrates also called rho-dipeptides (Patent Application FR 2 978 163). Alternatively only one of the reactive -NH groups is substituted with an arginine- or lysine-containing oligopeptide and the other -NH group is acetylated, so as to obtain Rho-monopeptides (Patent Application FR 2 978 164). In the patent applications mentioned, it is stressed that Rho-monopeptides, unlike Rho-dipeptides, do not neutralize heparin. However, the fact that they may have native fluorescence is not mentioned as an advantage or disadvantage.

Another complication in the determination of TG curves according to the established CAT technique is that during the experiment thrombin is bound to $\alpha$2macroglobulin so as to form $\alpha$2macroglobulin-thrombin ($\alpha$2MT), which is biologically inactive but does split small molecular weight substrates. This creates a parasitic thrombin like activity that has to be compensated for by methods known to the art. Large molecular weight synthetic substrates that are not split by $\alpha$2MT have been developed to circumvent this problem (International Patent Application WO 00/52199). Such substrates cannot be calibrated according to the CAT technique however because in that technique $\alpha$2MT has to be used as an external calibrator. The active center of $\alpha$2MT, however, is inaccessible to big substrates. The present invention eliminates the need to use $\alpha$2macroglobilin-thrombin as an external calibrator and therefore allows the use of substrates that are insensitive to $\alpha$2MT, hence avoiding the necessity of correcting for this parasitic activity as it arises in a clotting sample.

It is apparent from WO 2006/117246 as well as from WO 2007/141023 that developments made to date in the field of the invention do not avoid the recourse to a separate calibration experiment performed on a composition distinct from the assayed sample, for attempting to obtain an accurate measurement of thrombin and/or plasmin activity in a biological sample. There is thus still a need to provide TG assay conditions that would enable accurate measurement while using one sample only and thus being more practical for routine analysis. Additionally, while minimizing the amount of experiments to be run per sample, there is also a need for a method that would enable diminishing the volume and/or quantity of sample needed per analysis.

## Summary of the invention

The disclosure provides means, in particular kits, fluorogenic substrates and devices, and methods for the detection of hemostasis, coagulation and clotting disorders in biological samples comprising blood or blood products, such as whole blood and plasma samples. The invention is defined by the appended claims. The invention more particularly relates to an *in vitro* method for determining the activity over time of at least one proteolytic enzyme, in particular an amidolytic enzyme, in particular thrombin and/or plasmin, in a biological sample, especially a sample of plasma or whole blood, by assaying the conversion of fluorogenic substrate(s) by said proteolytic enzyme(s) over time. According to the invention, the activity over time of enzyme(s) is investigated by establishing a proteolytic enzyme generation over time curve, which is aimed at representing the generation and/or disappearance over time, and for an assayed sample, of proteolytic enzymes such as thrombin and/or plasmin transiently present in said sample, by measuring, as a physical parameter (fluorescence emission intensity) which can mathematically be further transformed into proteolytic enzyme concentrations or other transformed values having significance for an enzymologist or a biologist, generally after being also mathematically corrected for various influences, e.g. having being subjected to mathematical corrections for diminishing or suppressing the effect(s) of the influence of various factors or biases, such as attempted when performing a calibration process. Such generation over time curves can also be referred to as activity curves or curves illustrating the time course of thrombin and/or plasmin activity, and can be represented as a graphical representation of a measurement aimed at showing the development (generation and/or disappearance) of said proteolytic enzyme in a biological sample subject to an ongoing clotting phenomenon.

The disclosure also relates to means enabling the realization of such measurements, such as fluorogenic substrates as such and bound to a carrier such as (fluidic) chip.

The disclosure can be used for diagnosis purposes and is also directed to the use of the method of the invention for the detection or the monitoring of a condition of a patient, including for detection or monitoring of a pathological condition related to blood coagulation deficiency, or detecting a thrombosis or bleeding risk.

The disclosure also concerns the use of the method of the invention for the screening of substances, including for the screening of medications that could interact with the coagulation process, especially with thrombin or plasmin activity. Accordingly, the disclosure may also be used for the determination of the activity of pharmaceutical agents or drugs on the process of coagulation and fibrin clot formation, or for the assessment of activity of drug candidates in such process either by adding such drugs to blood or plasma samples during the experiment or by testing the blood or plasma from a healthy person, patient or experimental animal to which the drug has been administered.

The disclosure further relates to therapy monitoring of a patient, especially when said patient is under drug treatment.

## Description of the figures

**Fig. 1** *The features of a thrombin generation curve*

**Fig. 2** *A typical experimental thrombin generation curve*

Dotted line: fluorescent signal; drawn line: thrombin generation curve derived therefrom

**Fig. 3** *A typical experimental plasmin generation curve*

**Fig. 4** *The structure of a Rhodamine 110 based thrombin substrate*

**Fig. 5** *Diminution of experimental scatter through ratio calculation.*

Raw fluorescence data of a thrombin generation experiment; A: 300 $\mu$M mPEG-Gly-Pro-Arg-RhoAc; B: same after subtraction of initial fluorescence level; C: the same after division throug the initial fluorescence level, i.e. the ratio calculation. D: 75$\mu$M of mPEG-Gly-Pro-Arg-RhoAc; E: same after subtraction of initial fluorescence level; F: the same after division throug the initial fluorescence level, i.e. the ratio calculation.

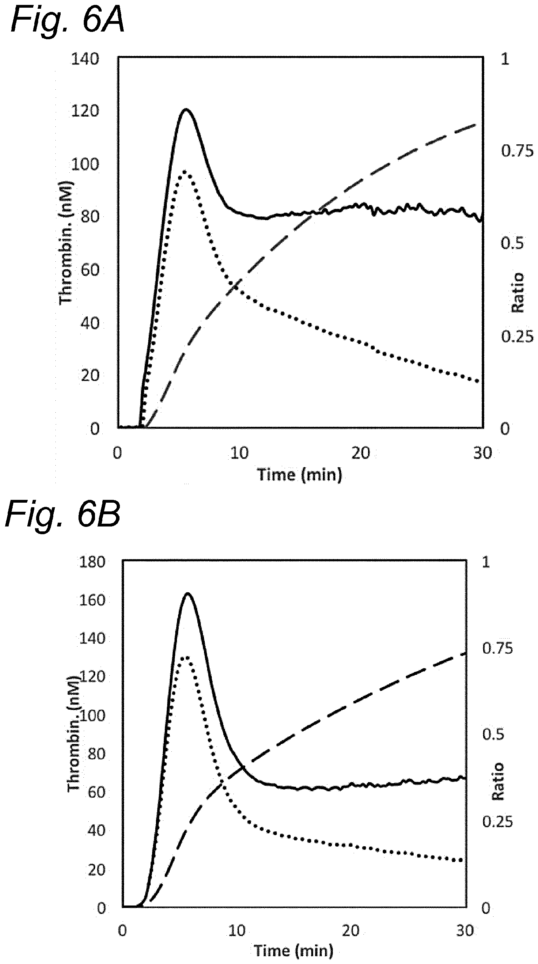

**Fig. 6** *The conversion of the normalized ratio curve into a thrombin activity curve.*

A Thrombin generation in defibrinated plasma after triggering with 5 pM tissue factor, using 108 $\mu$M Z-Gly-Gly-Arg-AMC as a substrate. Dashed line: Course of the normalized ratio. Dotted line: first derivative of the normalized ratio. Drawn line calculated from the previous traces using formula A. B The same as B with using 416 $\mu$M Z-Gly-Gly-Arg-AMC as a substrate.

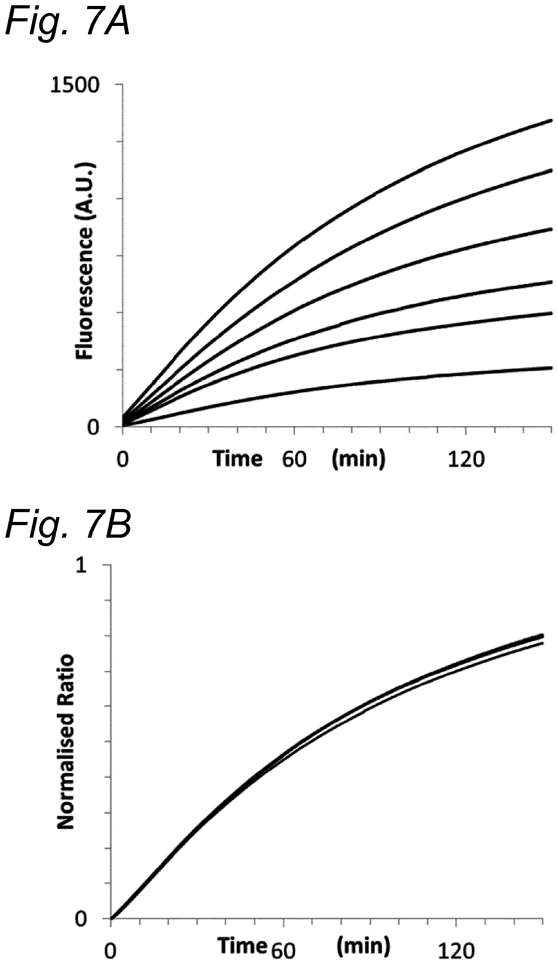

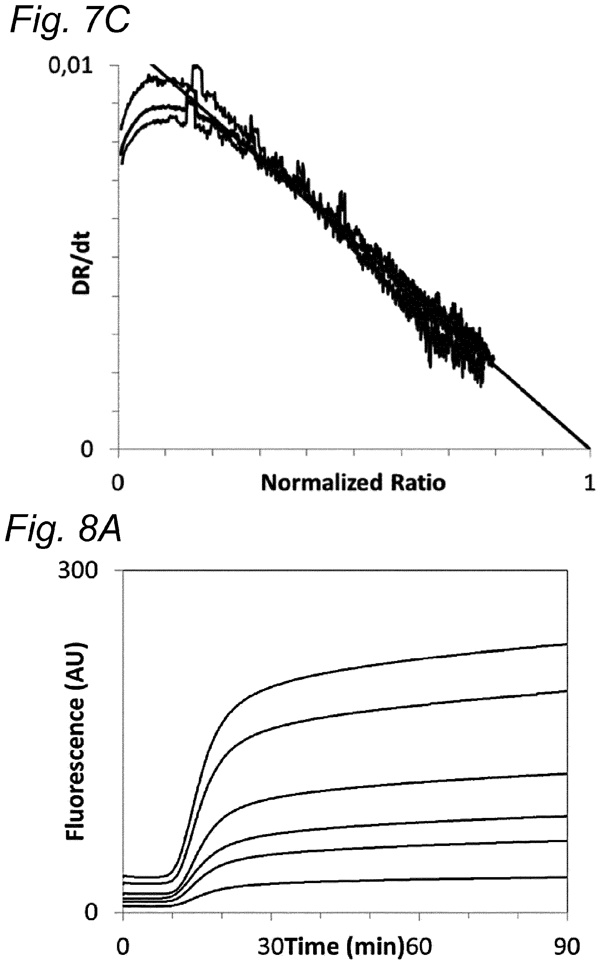

**Fig. 7** *Determination of the conversion factor of normalised ratio into thrombin concentration at substrate concentrations << $K_m$.*

Substrate: mPEG-Gly-Pro-Arg-RhoAc at concentrations of 75, 150, 225, 300, 450 and 600 $\mu$M. Frame A: original curves; Frame B: Normalized ratio; Frame C: Plot of dNR/dt against R.

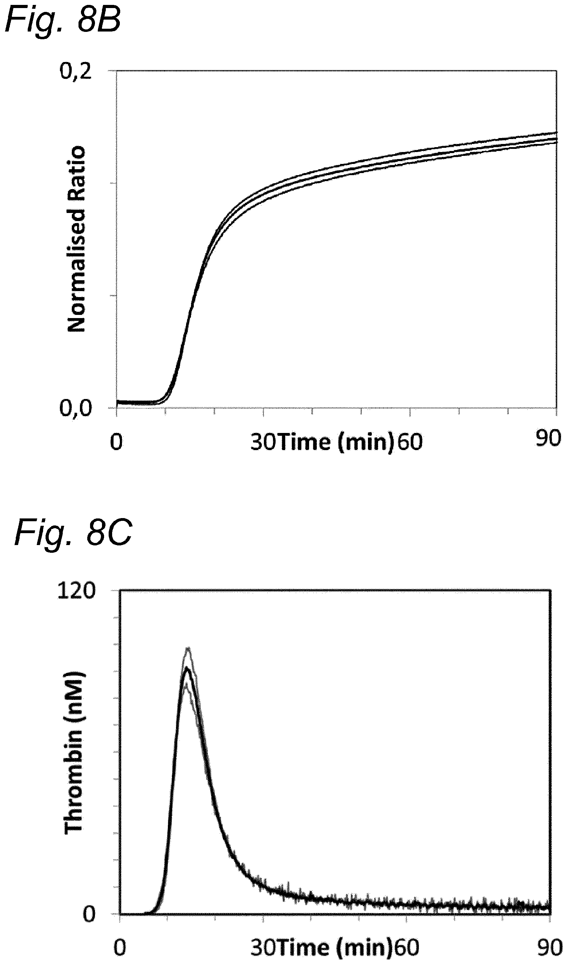

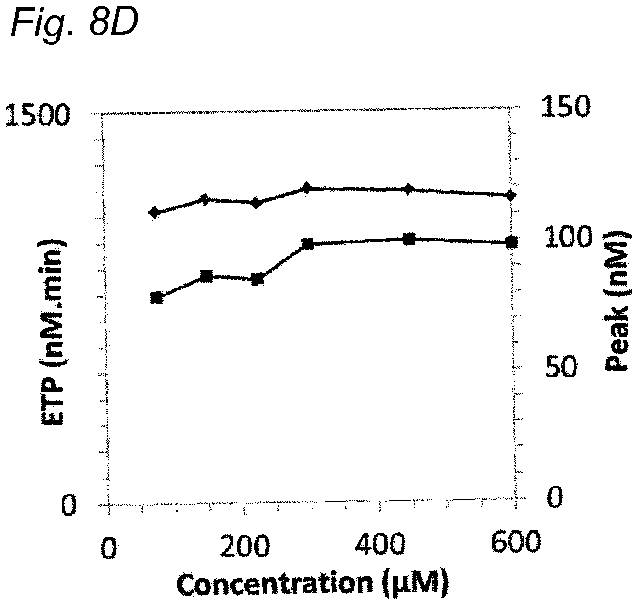

**Fig. 8** *Thrombin generation determined in filter paper.*

Substrate: mPEG-Gly-Pro-Arg-RhoAc at 75, 150, 225, 300, 450 and 600 $\mu$M.

Frame A: fluorescence curves; frame B: Normalized ratios; frame C: Thrombin generation curves as derived from the ratios; frame D: Values of ETP (triangles) and peak (circles) obtained at these different substrate concentrations.

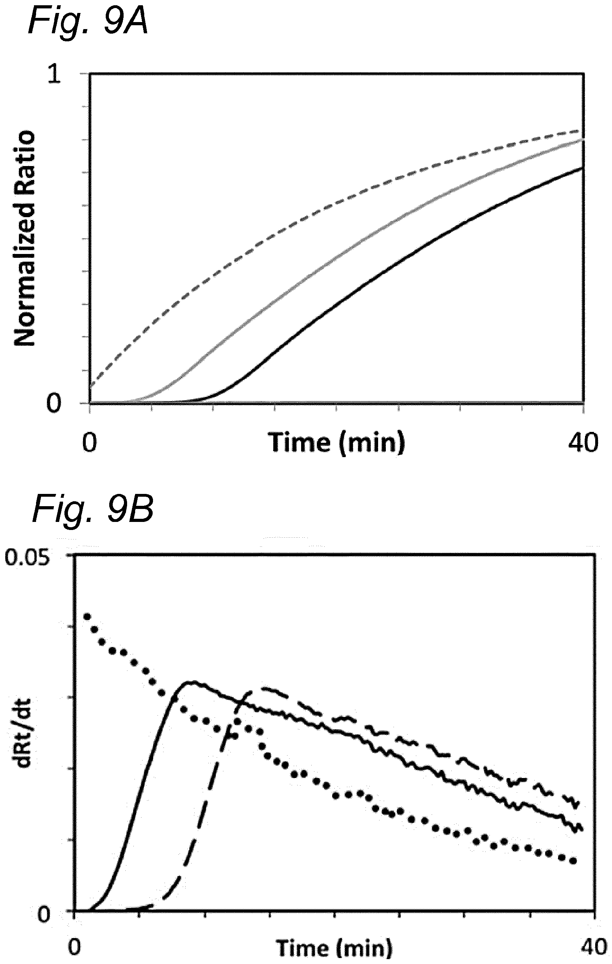

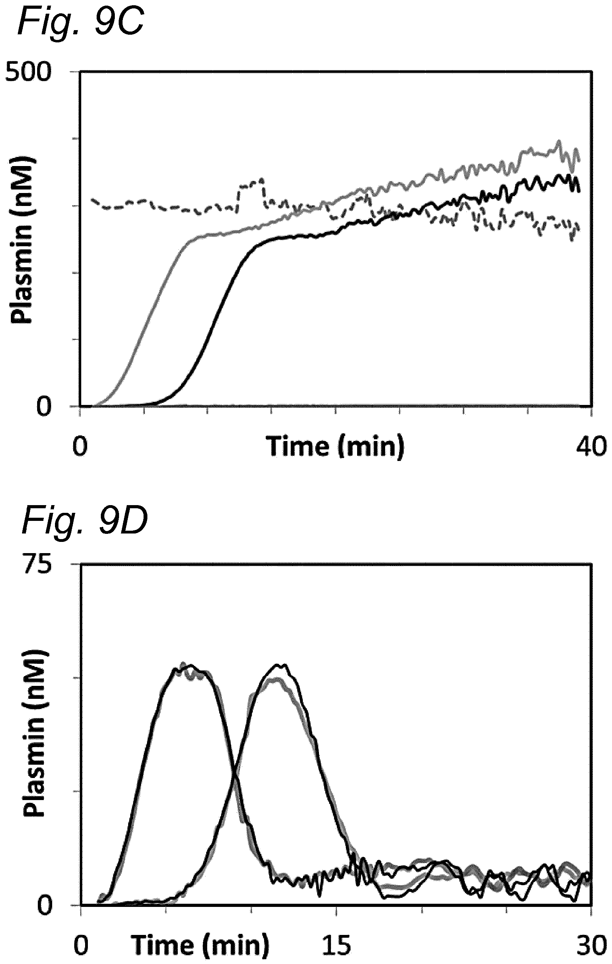

**Fig. 9** *A plasmin generation experiment evaluated with the ratio method.*

Frame A: Normalized ratio obtained from the raw fluorescence data at two tissue factor concentrations (drawn lines) and a calibrator (dotted line); frame B: first derivatives. Frame C: calculated total plasmin like activity; frame D: free plasmin activity: black lines: ratio method, grey lines standard method for comparison.

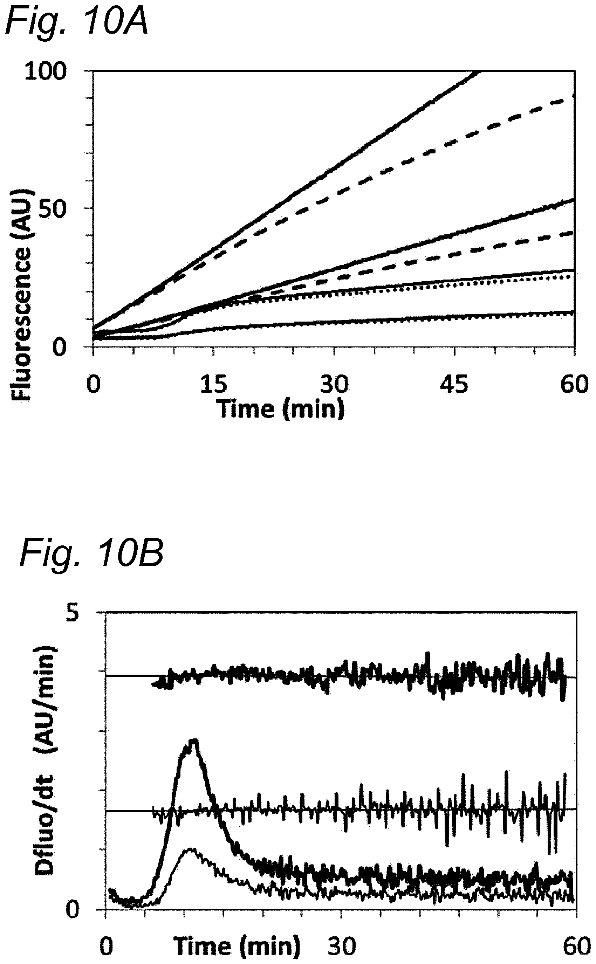

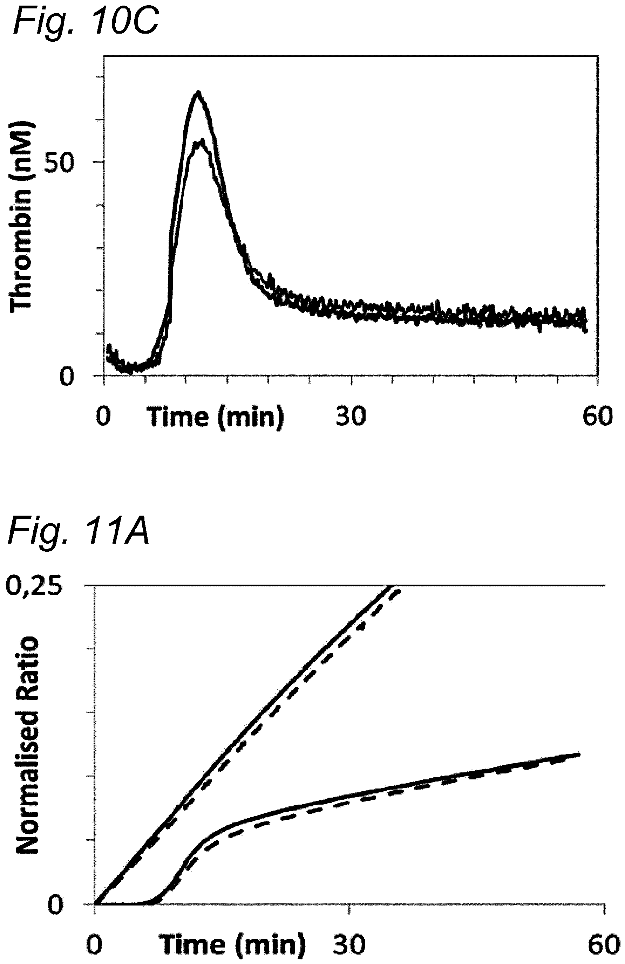

**Fig. 10** *Whole blood TG experiment. External calibration.*

Gly-Pro-Arg-RhoAc at 100 and 200 $\mu$M, 5 pM tissue factor.

Frame A: Dashed lines: raw fluorescence data: calibrator at 100 nM $\alpha$2M-thrombin; Dotted lines: thrombin generation experiment at the two substrate concentrations. Drawn lines: The same after correction for substrate consumption.

Frame B: first derivatives of the data in frame A after correction for substrate consumption. Frame C: Thrombin generation curves obtained from the adjacent frame by comparing to the calibrators according to the methods known to the art.

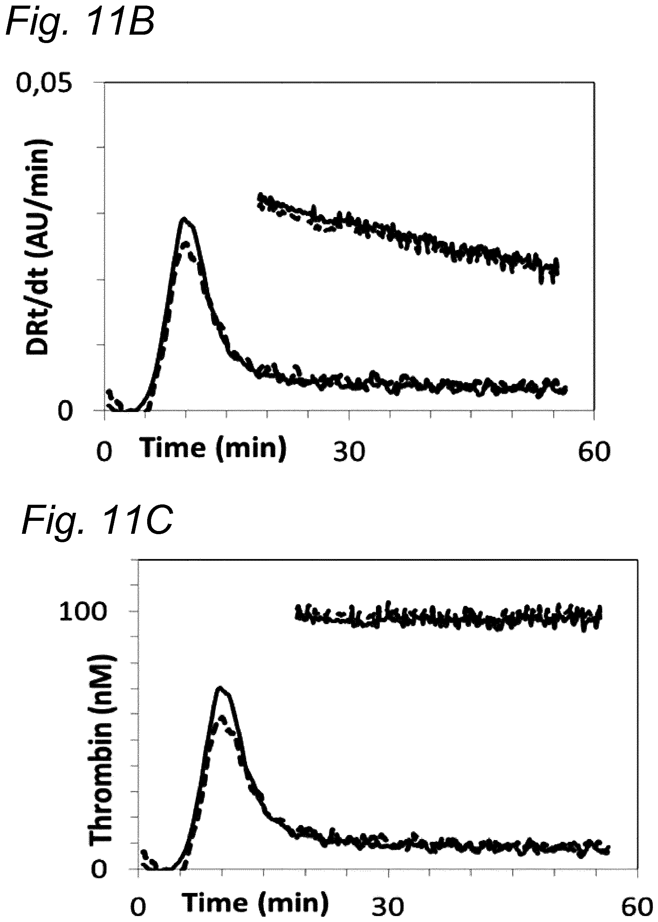

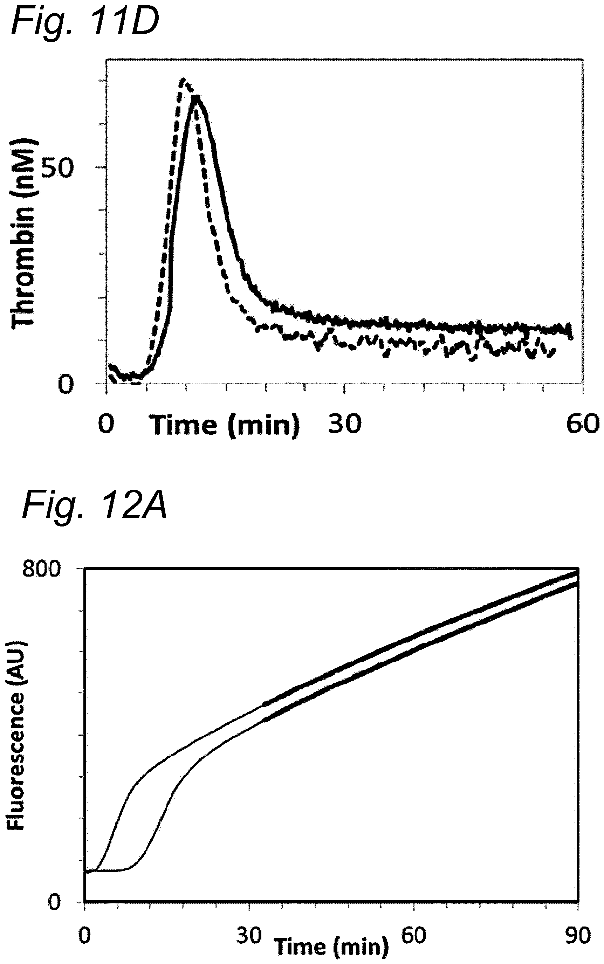

**Fig. 11** *Whole blood thrombin generation experiment, Ratio method.*

Same experiment as Fig. 10. Frame A: Normalized ratios at substrate concentrations 100 and 200 $\mu$M. Frame B: First derivative of the normalised ratio. Frame C: Thrombin concentrations as calculated from the two left frames. Frame D: Comparison of the calibrator (drawn)- and the ratio (dotted) method.

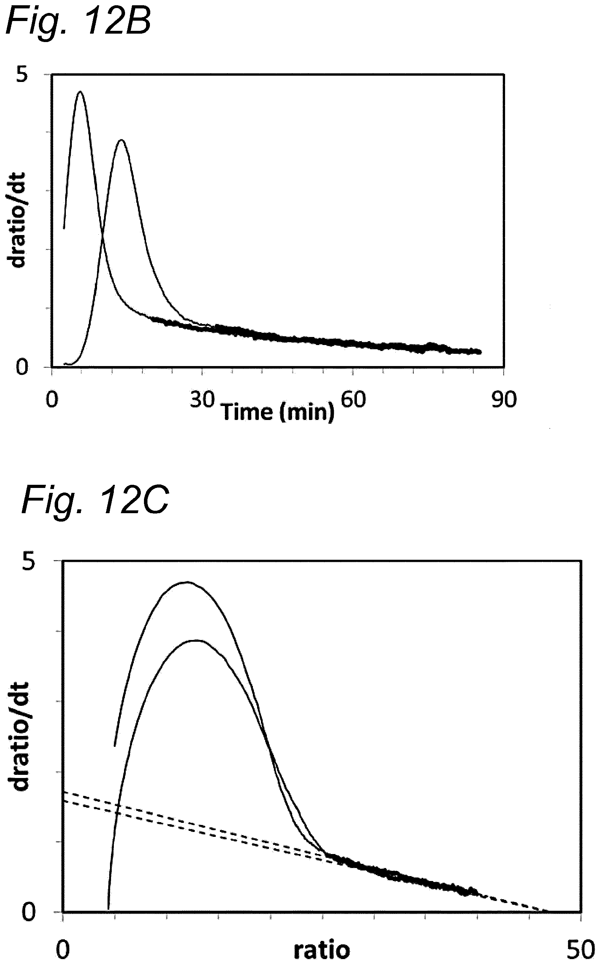

**Fig. 12** *Determining p from a TG experiment.*

Frame A: Fluorescence curves of a TG experiment with Z-Gly-Gly-Arg-AMC, 417 $\mu$M, as a substrate to which 1 $\mu$M AMC was added, triggered with 1 and 5 pM TF in normal pool plasma. Frame B: First derivatives of normalized ratios as a function of time. Frame C: First derivatives of normalized ratios as a function the normalized ratio. The bold part of the curve is the part in which no free thrombin is left. The dotted lines are linear regressions through that part; averages of three curves.

## Detailed description of the invention

The invention provides a method that overcomes the drawbacks of the known prior art methods. For this reason, the method of the invention is considered to be a self-calibrated assay to be used in thrombin and/or plasmin generation experiments. An important aspect of the invention, with respect to the methods known to date, is that no further compound having the mere function of an internal calibrator has necessarily to be added because the signalling substrate itself and/or its splitting product, serves as such internal calibrator. By the same token it allows measuring the effect of reversible direct thrombin inhibitors, unlike other methods in which the external calibrator and the experiment itself are both affected by the inhibitor.

The invention also allows the use of substrates that are not converted by α2-macroglobulin-thrombin complexes. Such complexes are used in all relevant prior art methods as calibrators. Because α2-macroglobulin-thrombin complexes form during thrombin generation, their activity in prior art methods has to be subtracted by calculation. This extra source of error is avoided by the present invention since it allows calibration even when macromolecular substrates insensitive to α2-macroglobulin-thrombin are used.

The method of the invention herein allows *in vitro* monitoring of the concentration of an enzyme as it appears in and disappears from a single biological sample due to a physiological process triggered in that sample. Like in prior art methods, the activity of the proteolytic enzyme, like thrombin and plasmin, is probed by its action on a fluorogenic substrate that is added to the sample. Unlike in the prior art methods, a fluorogenic substrate - or a mixture of fluorogenic substrate and fluorescent product - is used that has fluorescence of its own ("native fluorescence") when not split which increases upon splitting by the proteolytic enzyme. The concentration of the enzyme can therefore be calculated directly from the ratio of the developing fluorescence over the native fluorescence. This has the advantage that, unlike prior art methods, no separate calibration experiment needs to be run in parallel.

Accordingly, the invention provides a method for *in vitro* determination of the activity over time of at least one proteolytic enzyme in a biological sample, preferably a plasma sample or a whole blood sample, comprising determining over time the conversion of a fluorogenic substrate specific for said proteolytic enzyme, said method comprising the steps of:

a) contacting the sample with at least one fluorogenic substrate for a proteolytic enzyme wherein said fluorogenic substrate has native (zero-time) fluorescence and measuring the native (i.e. zero-time) fluorescence $F_0$, and contacting said sample with an agent triggering the generation of said proteolytic enzyme within the sample;
[Alternatively, when the substrate has no native fluorescence, zero time fluorescence can be obtained by adding a known amount of product ($P_0$), typically 1 - 10 mole% of the initial substrate concentration ($S_0$). Alternatively, when the generation of the enzyme does not start immediately after addition of the triggering agent - which is commonly the case - $F_0$ can be measured during step b) in the time lapse before enzyme generation starts (the so called lag time)]

b) generating a fluorescent signal over time curve by measuring, over time while said proteolytic enzyme is generated within the sample, the time course of at least one experimental fluorescence signal $F_{exp}.t$ emitted from the sample as a result of the release of a fluorescent group of the fluorogenic substrate specific for said proteolytic enzyme upon enzymatic action of the generated proteolytic enzyme on said fluorogenic substrate;

c) measuring in the same or in a similar sample, $F_{max}$ the maximal fluorescence that is obtained when all substrate has been converted into fluorescent product;
[Alternatively, the ratio $M=F_{max}/F_0$ may be determined in an independent experiment and provided to the end user because this ratio is dependent upon the ratio of the fluorescence yield of the product molecule over that of the substrate molecule and therefore a property of these molecules of a robust nature, i.e. a constant invariable for any substrate-product pair in a given reaction medium and that can be provided to the end user. When the substrate has no native fluorescence and the zero time fluorescence is obtained by adding a known amount of product, the ratio of the concentrations ($P_0/S_0$) equals $F_{max}/F_0$, which value again can be provided to the end user]

d) generating a normalized ratio over time curve by converting the values of $F_{exp}.t$ measured in step b) to values defined as the normalized ratio values $R_t$ according to the equation $R = (F_{exp}-F_0)/(F_{max}-F_0)$, wherein $F_0$ is the value of initial fluorescence measured in step a or during the lag-time in step b), $F_{exp}$ is the fluorescence measured over time in step b and $F_{max}$ is the fluorescence measured in step c or calculated from $F_0$ and the ratio $F_{max}/F_0$ as explained in step c;

[Thus $F_{exp.t}$ as a function of time ($F_{exp.t}$=f(t)) is converted into $R_t$ as a function of time ($R_t$=g(t))]

e) generating a proteolytic enzyme generation over time curve by applying the formula $E_t$=d$R_t$/dt.($S_0$+$K_m$/(1-$R_t$))/($k_{cat}$), wherein $S_0$ is the initial substrate concentration and $K_m$ and $k_{cat}$ are kinetic constants that govern the interaction between the proteolytic enzyme and the fluorogenic substrate.

These constants ($K_m$ and $k_{cat}$) are robust constants, invariable for any enzyme-substrate pair in a given reaction medium. Thus $R_t$ ($R_t$=g(t)) as a function of time is converted into $E_t$ as a function of time ($E_t$=h(t)).

Said method is herein referred to as the method according to the invention or the ratio method according to the invention.

*DEFINITIONS AND RELATIONS BETWEEN DEFINED VARIABLES*

A "**substrate**" is a molecule with a scissile bond (for example next to an arginine or lysine residue) that can be split by the measured enzyme (usually thrombin or plasmin). $S_0$ is the amount of substrate present at the start of the experiment, $S_t$ is the amount of substrate present at a moment **t** during the experiment

A "**product**" is a part of the substrate molecule that results after its splitting by the enzyme. More particularly it means the part that has fluorescent or light adsorbing properties and that thus can be used to monitor the course of the enzyme-substrate reaction. $P_0$ is the amount of substrate present at the start of the experiment, $P_t$ is the amount of substrate present at a moment **t** during the experiment.

"**Quantum yield**" is the percentage of adsorbed light energy that a fluorescent molecule renders as emitted light. The specific fluorescence $\Phi$ is the quantum yield per mole of the fluorescent molecule. Therefore $\Phi_S$ is the specific fluorescence of the substrate, and $\Phi_P$ that of the product. The actual values of $\Phi_S$ and $P_t$ are of no consequence as will be seen below, only the increase of $\Phi_p$ over $\Phi_s$ counts (see below).

$F_0$ is the fluorescence of the reaction mixture at zero time and during the lag time, as further defined hereafter, i.e. as long as no enzymatic activity has taken place. It is due to the own, especially native, fluorescence of the substrate, and to the fluorescence of the product in so far as it is present in the reaction mixture at zero time ($P_0$) So $F_0$=$S_0$.$\Phi_S$ + $P_0$.$\Phi_P$

$F_{exp}$ is the fluorescence of the reaction mixture due to the simultaneous presence of substrate and product, as this occurs during the process in which substrate is converted into product; $F_{exp.t}$ is $F_{exp}$ at moment t of that process. So $F_t$=$S_t$.$\Phi_S$ + $P_t$.$\Phi_P$ = ($S_0$-$P_t$)$\Phi_S$ + $P_t$.$\Phi_P$.

$F_{max}$ is the fluorescence of the reaction mixture when all the substrate has been converted into product, i.e. it is the fluorescence intensity that is measured by the end user when the reaction has run to completion. $F_{max}$=($S_0$+$P_0$)$\Phi_P$

**M** is the ratio of the fluorescence measured when all the substrate has been converted into product over the initial fluorescence when no substrate has been converted into product i.e. ($F_{max}$/$F_0$)-1. It is of no consequence whether the initial fluorescence is due to intrinsic fluorescence of the substrate or to an amount ($P_0$) present before the reaction starts. Addition of a small amount (0.1 - 10 mole%) of product to the initial reaction mixture can be used on purpose to apply the method of the invention to substrates without intrinsic fluorescence.

The fluorescence ratio **r** is the ratio of the increase of the experimental fluorescence value ($F_t$) obtained in the course of the experiment over the initial fluorescence value ($F_0$), so $r_t$=($F_t$/$F_0$)-1. $r_t$ is r at the moment **t** of the experiment. The normalised fluorescence ratio **R** referred to herein is $R_t$=($F_{exp.t}$-$F_0$)/($F_{max}$-$F_0$). $R_t$ is R at the moment **t** of the experiment. Alternatively $R_t$ can be calculated via the equivalent formula $R_t$=$r_t$/M. Rearrangement of the above formulas shows that $R_t$=($P_t$-$P_0$)/$S_0$. In words:

*The normalized ratio represents the fraction of substrate that has been converted in the course of an experiment.*

$k_{cat}$ is the number of molecules of substrate split per minute by the enzyme when saturated with substrate, and is a standard definition known in the art.

$K_m$ is the concentration of substrate at which the enzyme is half saturated, and is a standard definition known to in the art.

The "**lag-time**" is the part of the signal curve after the start of the reaction during which no significant product formation can be detected.

"**Surface fluorescence**" is the fluorescence that is observed when fluorescent material is dispersed on or in an opaque matrix, as further discussed hereafter. In the context of this disclosure, an opaque matrix may e.g. be porous cellulose, i.e. paper.

According to an embodiment, the proteolytic enzyme, the activity of which is assayed is thrombin, plasmin, or both.

*GRAPHIC REPRESENTATIONS (PLOT(S))*

The method of the invention is directed to determining the activity over time of at least one proteolytic enzyme.

A manner to evaluate such an activity is to collect data reflecting said activity over time. Such data may generally be graphically represented.

The graphic representations (plots) that may be obtained upon implementation of the method of the invention described herein encompass several types of plots, which are all considered as being part of the invention, since they can all be derived from the raw data collected upon release of the fluorescent group(s) of the fluorogenic substrate(s), as described herein.

A **"signal curve"** is a trace of the signal, typically fluorescence emission intensities (e.g. $F_{exp}$ values), over time (also named raw data or raw signal herein) that emerges from the sample in the course of time due to the formation of product from substrate by the enzymes(s) under study. (e.g. Fig. 2). Fluorescence intensities are usually expressed in Arbitrary Units (A.U.).

Such signal curves may be corrected for the influence of most experimental conditions by division through $F_0$, because $F_{exp}$ and $F_0$ are influenced in an identical way by experimental circumstances, so that these influences are equaled out by division.

If the experimental setup used for the registration of fluorescence intensities is subject to the so-called inner filter effect, leading to non-linearity between fluorescence and product concentration, this effect should be compensated for by methods know to the art.

Curves also may derive from a representation of the first derivative of corrected or non-corrected fluorescence emission intensities (e.g. dFexp/dt), physically representing a velocity of change of fluorescence plotted against time.

A **"Normalized ratio over time curve"** as specified in step c) above, can be obtained by converting the values of **Fexp** measured in step b) to values defined as a plot against time of the normalized ratio values $R_t$ calculated according to the equations developed in the previous section.

A **"Proteolytic enzyme** (usually thrombin or plasmin) **activity over time curve"** is a representation of an enzyme concentration plotted against time, also referred to as an **"thrombin generation curve** (e.g. Figs 1 and 2) or **plasmin generation curve"** (e.g. Fig. 3) herein. (e.g. Figs 1 and 2). An activity curve can be derived from a signal curve by comparison to a calibrator curve as in previous art, or directly from the signal curve through calculation of the normalize ratio as in the present invention.

As defined herein, "measuring" fluorescence values encompasses recording through an appropriate device and/or plotting such values on an appropriate support after gathering said fluorescence values.

As defined herein, "providing" a curve encompasses visualizing and/or plotting such a curve with an appropriate device or on an appropriate support.

*FUNCTIONAL NATURE OF SUBSTRATE(S) WITH RESPECT TO $F_0$ AND DETERMINATION OF $F_0$ AND $F_{max}$*

Substrates are known that possess a significant native fluorescence before being split by the enzyme. This leads to a non-zero fluorescence ($F_0$) at the start of the experiment. This is commonly seen as a drawback because the development of product is measured from the increase of that pre-existing native fluorescence, which consequently must be subtracted. It was surprisingly found that, when the native fluorescence is not subtracted but is used to express the progress of the reaction as the ratio (r) of the increase of fluorescence ($F_{exp}$) relative to the native fluorescence ($F_0$) then in the first place experimental variability is strongly reduced and in the second place the ratio can be used to determine enzyme activities without having to compare to a standard enzyme activity in a second sample.

To substrates that do not possess a significant native fluorescence a small amount ($P_0$, typically equal to 0.1% - 10% of $S_0$, preferably between 0.5% to 2% of $S_0$) of fluorescent product can be added to the initial reaction mixture before the reaction starts so as to enable the same calculations as with substrates that do possess native fluorescence.

The ratio of the fluorescence increase $r_{exp}=((F_{exp.}/F_0) - 1)$ is profitably normalised relative to the maximal possible ratio, $r_{max}=((F_{max.}/F_0) - 1)$, where $F_{max}$ is the fluorescence that is obtained when all substrate has been converted into product.

A normalised ratio is defined as $R=(F_{exp.}-F_0)/(F_{max.}-F_0)$.

In a given reaction medium, such as e.g. blood or plasma, $F_{max.}/F_0$ is a fixed number that is dependent upon the properties of the substrate and/or the amount of product present at the start of the experiment ($F_0$). This value is invariant and robust. It can either be determined by the end user or provided to the end-user together with the reagents necessary for carrying out the experiment, thereby $F_{max}$ can be calculated from the measured value of $F_0$.

In step b), starting the recording of fluorescence can be made either before or after contacting the sample with reagents that trigger the formation of the proteolytic enzyme under study (step a). The nature of these agents is discussed hereafter.

According to a particular embodiment, $F_0$ is previously determined and provided to the user of the method.

Alternatively, the user may determine the value of $F_0$ when implementing the method of the invention. $F_0$ can in particular be determined (measured) during step b). In this case, measuring the value of native fluorescence ($F_0$) can be made either before enzyme formation is triggered or during the lag time of the reaction.

According to an embodiment, when the assayed proteolytic enzyme is thrombin, $F_0$ can be determined as being the fluorescence value during the lag time of thrombin generation measured in step b).

According to a particular embodiment, when the assayed proteolytic enzyme is plasmin, $F_0$ can be determined as being the fluorescence value during the lag time of plasmin formation, that, because plasmin formation as triggered by tissue plasminogen activator, is dependent upon the presence of fibrin, is equal to or longer than the lag time of thrombin generation.

The term "lag time" as used herein is meant to indicate the time it takes before thrombin formation really starts, i.e., becomes detected. The concentration of thrombin typically starts at zero, rises to a peak height of usually a value between 50 and 500 nanomolar and goes back to zero again. The lag time usually lasts between 1 and 20 minutes and is finished as soon as the thrombin concentration is approximately above 5 nanomolar. At this moment also the formation of a clot appears, whereas the peak occurs a few minutes later. Plasmin generation triggered by tissue plasminogen activator (tPA) PA occurs after fibrin appears, so that the lag time of thrombin generation is also the time that can be used for the determination of the zero level of a plasmin substrate.

Limits of concentration to be used for the fluorogenic substrate may vary depending on the apparatus used for fluorescence measurement. Any apparatus (fluorometer) conventionally used in the field of the invention can be employed. The upper limit of fluorogenic substrate concentration is defined by the solubility of the substrate in the sample. The lower limit of substrate concentration is dependent upon the sensitivity and the background noise level of the fluorometer.

Preferably, a fluorogenic substrate in the assayed sample is used in concentrations ranging from 100 nM to 1 mM, preferably ranging from 100-500 $\mu$M, more preferably ranging from 50 $\mu$M to 500 $\mu$M. The fluorogenic substrate can be an oligopeptide having the sequence of 1 to 30, preferably 2 to 30, more preferably 3 to 20, more preferably 3 to 10, more preferably 3 to 5, such as 3, 4 and 5 amino acid residues coupled with a fluorescent molecule or any other molecule that renders a fluorescent signal upon cleavage by a proteolytic enzyme to be assayed. The oligopeptide preferably has a terminal lysine or terminal arginine coupled to the fluorescent molecule

*ENZYME ACTIVITY CURVE*

Converting signal curve(s) into enzyme activity curve((s) can be made by first converting the values of $F_{exp}$ measured in step c) above to values defined as normalized ratio values $R$ as defined above. As explained above, the normalized ratio equals the ratio of the amount of product formed over the original substrate concentration. During the reaction $F_{exp}$ increases as an (unknown) function of time so $(F_{exp.t}=f(t))$, consequently $R$ also increases as a function of time $(R_t=f(t))$.

According to classical enzyme kinetics as known in the art, the reaction velocity at any moment t during the course of a reaction is $v_t = dP_t/d_t = k_{cat}.E_t.S_t/(K_m+S_t)$ where $K_m$ and $k_{cat}$ are respectively the Michaelis constant and the turnover constant of the enzymatic reaction, $E_t$ is the enzyme concentration, $P_t$ the product concentration and $S_t$ the substrate concentration, the subscript $t$ indicating that these variables change in time.

Substituting $P_t=S_0.R_t$ and $S_t=(1-R_t).S_0$ then gives $S_0.dR_t/dt=k_{cat}E_t(1-R_t)/(K_m/S_0+(1-R_t))$, by rearrangement it follows that:

$$E_t=dR_t/dt.(S_0+K_m/(1-R_t))/(k_{cat}) \qquad \textbf{Formula A}$$

Wherein $K_m$ and $k_{cat}$ are the kinetic constants of the enzyme-substrate reaction involved, $S_0$ is the initial substrate concentration. This formula allows to calculate the course of enzyme activity ($Et=f(t)$) from the course of the normalised ratio ($Rt=f(t)$) when only the reaction constants $K_m$ and $k_{cat}$, are known.

It should be noted that the relation between $E_t$ and $R_t$ becomes independent of $S_0$ for $S_0 << K_m$. In the section "examples" we will encounter such cases. In that case formula A reduces to

$$E_t=dR_t/dt/(1-R_t).(K_m/k_{cat}) \qquad \textbf{Formula A'}$$

This formula also allows to test whether the condition $S_0 << K_m$ applies. Because it predicts that at constant E there is a linear relation between dRt/dt and $Rt$. Viz:

$$dR_t/dt = (E_t . k_{cat}/K_m) - R_t . (E_t \; k_{cat}/K_m)$$

Therefore, according to an embodiment, the present invention is directed to a method according to the above, comprising a step of determining the course of the concentration of thrombin and/or plasmin in a biological sample over time, wherein one of the proteolytic enzymes is respectively thrombin and/or plasmin and the resulting proteolytic enzyme generation over time curve is respectively a thrombin and/or plasmin concentration over time curve, which is determined by converting the values of $F_{exp}$ measured in step b) first to normalized ratio values and then to thrombin and/or plasmin concentration values using the formulas developed above.

An advantage of this particular embodiment wherein the concentration of thrombin and/or plasmin is determined, is that, contrary to prior art, no correction for substrate consumption is required.

$K_m$ and $k_{cat}$ are robust constants known to the art, invariable for a given enzyme-substrate pair and given reaction conditions. They can therefore be provided to the end user.In an embodiment of the present invention the enzyme is thrombin and the reaction conditions imply a medium consisting of plasma diluted between 2:3 and 1:10. The medium therefore contains high concentrations of antithrombins so that added thrombin is rapidly inactivated.

For determination of the kinetic constants variants of thrombin and plasmin have to be used that do not interact with antithrombin or antiplasmin. To this end, thrombin and plasmin can be reacted with the plasma-derived protein $\alpha$2-macroglobulin. Antithrombin-resistant thrombin activity can also be obtained reacting prothrombin with the protein staphylocoagulase. The resulting $\alpha$2-macroglobulin-thrombin, $\alpha$2-macroglobulin-plasmin and staphylocoagulase-thrombin complexes retain their enzymatic activities towards chromogenic and fluorogenic substrates unless the molecular weight of such substrates is > 5 kD, but are immune to protease inhibitors. As it is not sure that the kinetic constants of the complexes are exactly identical to those of the free enzyme, the resulting values should be validated. To this end in a standard or reference plasma the thrombin forming capacity should be accurately determined by a method known to the art, e.g. by calibrated automated thrombinography. Then the method here disclosed should be applied and the values of $K_m$ and $k_{cat}$ used should be fine-tuned so as to obtain the same thrombin generation curves with both methods. $K_m$ and $k_{cat}$ can thus be obtained that will be identical for all similar experiments and can be provided to the end user of the method of the invention. Alternatively, they can be(re)determined by the end user himself according to the above guidelines.

This way of calculation abolishes the need for running a separate calibration experiment and thus halves the experimental effort and diminishes the experimental error. Therefore, such a method of the invention can also be considered as a method enabling the realization of a novel way of calibration, which requires only one sample to be assayed.

A large number of experimental variables such as variables in the measuring device, variables in the sample support and variables in substrate concentration and fluorescence quenching are identical in the native fluorescence and in the developing experimental fluorescence and thus equal out upon calculation of the normalised ratio, which again diminishes the experimental error.

## TYPE OF SAMPLE / TRIGGERING

In an embodiment, the assayed biological sample is a blood sample, optionally anticoagulated through addition of sodium citrate or the like, and is in particular a whole blood sample. A blood sample can be a reconstituted blood sample.

In an embodiment, the assayed biological sample is a plasma sample, such as a Platelet Poor Plasma (PPP) sample or a Platelet Rich Plasma (PRP) sample or a platelet-free plasma sample. In the methods according to the invention, when thrombin and/or plasmin are assayed, thrombin and/or plasmin are allowed to generate in the sample after contacting said sample with components suitable for triggering thrombin and/or plasmin generation.

Triggering agents for enabling the generation of thrombin and/or plasmin within the sample within the method of the invention can be one or several agents selected from the group consisting of: calcium ions, phospholipids, tissue factor, soluble tissue factor, thromboplastin, kaolin, elagic acid for triggering thrombin generation, and plasminogen activator, in particular tissue plasminogen activator (tPA), urokinase and streptokinase. Selected suitable agents may be merged within a single mixture before being put into contact with a sample to be assayed according to the method of the invention.

In an embodiment, Tissue Factor, possibly in the presence of calcium ions, in particular when a citrated blood sample is assayed, is used for triggering thrombin generation.

In another embodiment tissue plasminogen activator (tPA) is used for triggering plasmin generation. The triggering agents that can be put into contact with the sample in order to allow thrombin and/or plasmin generation are added in quantities enabling coagulation to start. Such quantities can be within the range of 0.05 picomole/L to 15 nanomole/L for tissue factor, and around 10 mM of $Ca^{++}$ ions when citrated blood is used. When native, non-anticoagulated blood is used, no $Ca^{++}$ ions need to be added. Alternatively, in certain applications it is profitable not to add tissue factor in order to investigate the spontaneous coagulability of the blood e.g. through the activity of microparticles. Tissue factor, when needed, is put into contact with the sample upon starting the measurement.

*MEASUREMENT OF SURFACE FLUORESCENCE/REACTION VESSEL VOLUME AND SHAPE /POROUS MATRIX*

It is one of the advantages of the method that samples of arbitrary size and shape can be utilized. The size and shape of the sample influences both the amount of fluorescent molecules that is exited as well as the amount of emitted light that is recovered by the detecting instrument. These influences are identical in the determination of the initial fluorescence ($F_0$) and fluorescence measured during the experiment ($F_{exp}$) and the maximal fluorescence ($F_{max}$) and therefore equal out upon determination of the (normalized) ratio.

According to an embodiment, step a) is performed by putting the sample in a porous opaque matrix. In this embodiment, fluorescence is measured in small pools of undefined depth on the surface of the porous matrix and of undefined form, leading to unknown effects of reflection and refraction. This type of fluorescence is also known to the art as surface fluorescence. It is known in the art that measuring surface fluorescence has the advantage that the inner filter effect is absent and therefore does not have to be corrected for. It is a disadvantage of measuring surface fluorescence that that the relation between fluorescence intensity and concentration of fluorophore remains unknown. Because the initial native fluorescence and the fluorescence that develops during the experiment originate from exactly the same fluid pools, the relative intensities of $F_0$, $F_{exp}$ and $F_{max}$, are independent of the absolute intensity. In other words, this disadvantage disappears when the ratio method according to the invention is used because by taking the ratio, influences of unknown optical effects equal out. Thus according to an embodiment, the biological sample is deposited in a container or on a support, such as a chip, preferably a microfluidic chip, said container or support having the fluorogenic substrate adsorbed on a solid opaque matrix placed within the container or adsorbed on the support.

*STRUCTURE OF THE SUBSTRATE(S) / FLUOROGENIC GROUP(S) / SIMULTANEOUS MEASUREMENT OF THROMBIN AND PLASMIN.*

In the methods according to the invention, the fluorogenic substrate preferably is a rhodamine-based fluorogenic substrate, preferably a rhodamine-based peptide, preferably encompassing a Rhodamine110 fluorophore, or is an AMC-based fluorogenic substrate, wherein, optionally, the sample is supplemented with an amount of the fluorescent product corresponding to the fluorogenic substrates or a different fluorescent product, equal to between 0.1% and 10% of the fluorogenic substrate in the sample.

Among the different possible fluorogenic groups, amino-methyl-coumarine (AMC) and Rhodamine 110 (Rho) are most commonly used. The latter deserves particular attention. The molecule (Fig. 4) contains two reactive groups of which one can be substituted by a polypeptide and the other by an acyl residue. Such Rho-monopeptides are described in Patent Application FR 2 978 164 without, however stressing the fact that such peptides already show a considerable native fluorescence when the peptide is not split off. The fact that they have a native fluorescence is not mentioned as an advantage or disadvantage in said patent application. It is precisely the native fluorescence that unexpectedly turned out to be exploitable as an advantage because it allows internal, especially "intra-molecular", calibration, which is the gist of the present application. The term monopeptide substrate herein means that that substrate comprises a single peptide and should not be construed as if the monopeptide would be a single amino acid.

According to an embodiment, the substrate contains Rhodamine 110 as a fluorophore and is coupled to an oligopeptide at one of the two reactive NH-groups on the Rhodamine 110 molecule (Fig. 4), the other reactive NH-group being coupled to an acetyl group or other group that does not completely quench the fluorescence of the Rhodamine 110 moiety (Rhodamine 110-based monopeptide-substrates). Such substrates have the advantage that they have a native fluorescence of their own. Preferably, the monopeptide is coupled to the Rhodamine 110 group at a terminal lysine or arginine group of the oligopeptide and/or the other reactive NH-group of the Rhodamine 110 group is acetylated. Preferably, the oligopeptide of the substrate has the amino-acid sequence: Gly-Gly-Arg, Gly-Pro-Arg, Ile-Pro-Arg, Phe-PIP-Arg, Val-Leu-Lys, Gly-Pro-Lys or Ala-Phe-Lys.

In another embodiment, the fluorogenic group Rhodamine 110 is coupled to two oligopeptides at the two reactive NH-groups of said Rhodamine 110 moiety, in which case the native fluorescence disappears, believed to being quenched by the peptides. This is also the case when a substrate carries 7-amino-4 methylcoumarin (AMC) as a fluorogenic group. According to the elements provided above, in this situation an amount of fluorescent product must be added to such a substrate in order to provide for the native fluorescence at $T_0$ in a method of the invention.

In the method according to the invention, it can be especially useful that the fluorogenic group is coupled to a terminal lysine or arginine residue of the oligopeptide because thrombin, like plasmin splits preferably groups bound to these amino acid residues, where thrombin has a preference for arginine residues and plasmin for lysine residues.

In an embodiment, at least one peptide of the fluorogenic substrate used for signaling thrombin generation comprises or consists of the amino-acid sequence: Gly-Gly-Arg or Gly-Pro-Arg. Within the scope of the invention, a peptide of the fluorogenic substrate used for determining thrombin or plasmin generation can have any amino-acid sequence, provided that it has a length of 1 to 30 amino-acid residues and an arginine- or lysine amino-acid residue coupled to the fluorescent molecule.

According to an embodiment, a fluorogenic substrate is used that is exclusively sensitive to either thrombin or to plasmin, the resulting method being an exclusive measurement of either thrombin or plasmin activity over time.

Alternatively, according to another embodiment, in the method according to the invention, thrombin and plasmin generation over time curves are simultaneously determined, either using a fluorogenic substrate that is sensitive to both enzymes or a mixture of a plasmin fluorogenic substrate and a thrombin fluorogenic substrate that yield the same fluorescent product.

It has been established by the inventors that a rhodamine-based monopeptide substrate having a bulky group chemically bound to the peptide part of a Rho- monopeptide is particularly advantageous for implementing the method of the invention. Examples of such substrates are given in Patent Application WO00/521 99. The bulky group prevents the substrate to be split by the reaction product of plasmatic $\alpha$2macroglobulin with thrombin ($\alpha$2M-T), which arises during a thrombin generation experiment and pertains in serum and causes residual enzymatic activity on small substrates after thrombin generation is over.

In the usual CAT method, such substrates cannot be used because $\alpha$2M-thrombin is employed there as the external reference enzyme. Because the present invention does not require an external reference, such substrates can be used with the present invention.

Coupling of a thrombin- or plasmin substrate with inert bulky carrier molecule(s) may be advantageous because, as is known from WO 00/52199, using such substrates, hydrolysis stops when all free thrombin and/or plasmin in a plasmin- or thrombin generation experiment has been inactivated. Hence the development of $\alpha$2M-thrombin and/or $\alpha$2M-plasmin complexes does not give rise to the parasitic activity that is observed when small substrates are used, and this activity has therefore not to be compensated for by calculation, and thus a source of error is abolished.

By "bulky group", it is meant a carrier molecule having as function to render the fluorogenic substrate less reactive to thrombin or plasmin that is bound to $\alpha_2$macroglobulin, in particular an inert carrier molecule larger than 5kD, more preferably larger than 10 kD, that may be, for example, a protein, a polysaccharide or a synthetic polymer. Preferably, the inert carrier molecules are hydrophilic so that they have a good solubility under the conditions of the experiment. Bulky substrates are not a prerequisite for the present invention but the present invention is a prerequisite for the use of bulky substrates in the establishment of a thrombin- or plasmin generation curve, i.e. to exploit the advantageous properties of such substrates. Preferably, the bulky group is polyethylene glycol (PEG), preferably having a Mr of at least 5kD, more preferably between 5kD and 35 kD.

According to an embodiment, a fluorogenic substrate according to the disclosure Rhodamine-based monopeptide as defined herein, the peptide of which is also coupled to a bulky hydrophilic group having a weight of at least 5 kD, such as a polyethylene glycol (PEG) group.

According to an embodiment, a fluorogenic substrate according to the disclosure suitable to be adsorbed on a solid porous matrix placed in a container or adsorbed on a support. Such an opaque matrix or support may be a cellulose-based support, such as a filter paper (see above the section regarding surface fluorescence).

The invention is particularly suitable for use for monitoring in "real time" thrombin and/or plasmin generation in a biological sample. The term "real time" as used herein is meant to indicate that the course of enzyme concentration in the assayed biological sample is displayed simultaneously with the ongoing appearance and/or disappearance of thrombin and/or plasmin in the assayed sample. The method of the invention can be advantageously used for *in vitro* detecting or monitoring a haemostatic disease or a thrombotic disease or for detecting or monitoring the possibility that such a disease occurs in a patient.

The method of the invention can also be advantageously used for monitoring the treatment of a haemostatic disease or a thrombotic disease by measuring the effect on thrombin generation of the administration of haemostatic or antithrombotic drugs.

The method also enables the *in vitro* detection or monitoring the interaction of candidate substance(s) on thrombin and/or plasmin activity in a whole blood sample, wherein said determined substance(s) is (are) added to the sample to be assayed or is (are) added during thrombin and/or plasmin generation.

Candidate substances that can be tested according to the method are for instance pharmaceuticals or other compounds having a coagulation effect on blood, such as coagulation factors or drugs or anticoagulant factors or drugs. Thrombin- and plasmin inhibitors, but also plasmin activators can especially be tested according to the method of the invention.

According to an embodiment of the invention, reversible thrombin inhibitors can be tested, in particular thrombin inhibitors that could not be tested through a method such as the CAT method, that are dependent upon the simultaneous measurement of a calibrator containing a thrombin like enzyme such as the $\alpha$2macroglobulin-thrombin complex, because such inhibitors inhibit calibrator and generated enzyme alike.

A particular useful embodiment of the invention is the use of bulky substrates for the above purpose the complications due to the action of the drug on the $\alpha$2macroglobulin-thrombin complex generated during the thrombin generation process are avoided (see further Wagenvoord RJ, Deinum J, Elg M, Hemker HC: The paradoxical stimulation by a reversible thrombin inhibitor of thrombin generation in plasma measured with thrombinography is caused by alpha-macroglobulin-thrombin. Journal of thrombosis and haemostasis : JTH 2010, 8:1281-9).

In another aspect, the method of the invention can be used to screen substances in order to determine their capacity of interaction with thrombin and/or plasmin activity.

According to the invention, the method which has been described above and which will be illustrated in the examples can be especially used for *in vitro* measurement of Endogenous Thrombin Potential (ETP), or Endogenous Plasmin Potential, especially in a whole blood sample, analogously the endogenous plasmin activity can be determined. The term "ETP", as used herein, means the time integral of the curve until the moment of (near)zero thrombin activity is attained.

It is also useful to measure *in vitro* the level of the peak(s) of thrombin and/or plasmin generated during the assay.

It can be also used for *in vitro* measurement of time to peak for thrombin and/or plasmin or for measurement of the lag times which is equivalent to the clotting time.

As used herein, the term "level of the peak of thrombin (c.q. plasmin)" means the maximal thrombin (plasmin) activity attained. The term "time to peak", as used herein, means the time between the start of the reaction and the moment that the peak is reached.

The invention further provides for a method of *in vitro* determining the activity over time of plasmin in a biological sample obtained from an individual, comprising the steps of:

i) performing the method according to the invention of *in vitro* determination of the activity over time of thrombin in said biological sample, wherein only thrombin generation is triggered, and
ii) performing the method according to the invention of *in vitro* determination of the activity over time of thrombin and plasmin in the biological sample analyzed in step i), wherein both thrombin and plasmin generation are triggered, and
iii) subtracting the output obtained in i) from the output obtained in ii) to obtain a representation of the activity over time of plasmin for the assayed sample.

The disclosure also provides a kit for carrying out the method of the invention, which comprises:

- at least one fluorogenic substrate according to the disclosure for a proteolytic enzyme, preferably thrombin and/or plasmin, that may be chosen from but is not restricted to the substrates mentioned in the examples, preferably a substrate comprising Aminomethylcoumarine or Rhodamine 110 as a fluorogenic group wherein the fluorescence is quenched by esterification to oligopeptides via the amino acids Arg or Lys;
- an agent(s) for triggering generation of a proteolytic enzyme, preferably for respectively triggering thrombin and/or plasmin generation, such as tissue factor and calcium ions to enable thrombin generation or tissue plasminogen activator to trigger plasmin generation;
- optionally, means for containing the reaction fluid such as a solid porous matrix to be placed in a container or made integral with a support;
- optionally, a container with means for adsorbing the fluorogenic substrate and/or, a support comprising the fluorogenic substrate adsorbed, preferably dried, on said support, said support being for example a chip, preferably a microfluidic chip.

Means for adsorbing the fluorogenic substrate may be a solid porous matrix to be placed in a container or made integral with a support. Such a matrix or support may be a cellulose-based support, such as a filter paper, and can suitably retain (adsorb) a fluorogenic substrate according to the definition provided herein.

According to an embodiment, the kit according to the disclosure also comprises agents interacting with proteolytic enzyme(s) generation, preferably thrombin and/or plasmin generation, an activator of thrombin and/or plasmin generation such as Russell's viper venom (two types), Echis Carinatus Venom, Activated protein C, Activators of protein C like thrombomodulin or Protac (From Agkistrodon contortix venom) or an inhibitor like corn trypsin inhibitor that prevents contact activation.

Optionally, the kit also comprises directions for use in order to provide specific guidance to carry out the method of the invention.

The disclosure also relates to the use of the above-mentioned reagents for manufacturing a kit for performing a method for determining *in vitro* proteolytic enzymes activity curves or for improving the signal over noise ratio of a method of *in vitro* determining the activity over time of at least one proteolytic enzyme in a biological sample, especially a plasma sample or a whole blood sample. The disclosure also provides a fluorogenic substrate for use in a method according to the invention, which is a Rhodamine-based monopeptide, wherein one of the symmetrical reactive NH-groups of Rhodamine is coupled to an oligopeptide through an arginine residue within the oligopeptide and the other reactive NH-group of the Rhodamine is acetylated and wherein the oligopeptide is coupled to a bulky hydrophilic group having a weight of at least 5 kD, such as a polyethylene glycol (PEG) group. Preferably, the bulky hydrophilic group is selected amongst polyethylene glycols, and unreactive proteins like albumin. Preferably, the oligopeptide comprises or consists of: Gly-Gly-Arg, Gly-Pro-Arg, Ile-Pro-Arg, Phe-PIP-Arg, Val-Leu-Lys, Gly-Pro-Lys or Ala-Phe-Lys.

The disclosure also relates to a fluorogenic substrate as defined herein. The disclosure also provides a carrier, preferably a chip, more preferably a microfluidic chip comprising the fluorogenic substrate according to the disclosure.

The disclosure also relates to an apparatus for carrying out steps b) and c) of the method according to the invention, and optionally for further carrying out the determination of the concentration of thrombin and/or plasmin in a biological sample over time according to the definitions provided herein (above).

The disclosure also relates to a computer readable medium comprising software code adapted to cause the above-defined apparatus to perform steps b) and c) according to the method of the invention as defined herein (above), and optionally for further carrying out the determination of the concentration of thrombin and/or plasmin in a biological sample over time according to the definitions provided herein (above). This software code enables carrying out the method of the invention when executed on the data processor of the above-defined apparatus. Parameters $K_m$, $k_{cat}$ and M as defined above may be provided for enabling the software code to cause the above-defined apparatus to perform said steps b) and c) or said determination of the concentration of thrombin and/or plasmin.

Other features and advantages of the invention will be disclosed in the following sections and in the figures.

In this document and in its claims, the verb "to comprise" and its conjugations is used in its nonlimiting sense to mean that items following the word are included, but items not specifically mentioned are not excluded. In addition, reference to an element by the indefinite article "a" or "an" does not exclude the possibility that more than one of the element is present, unless the context clearly requires that there be one and only one of the elements. The indefinite article "a" or "an" thus usually means "at least one".

The word "about" or "approximately" when used in association with a numerical value (e.g. about 10) preferably means that the value may be the given value (of 10) more or less 5% of the value. The sequence information as provided herein should not be so narrowly construed as to require inclusion of erroneously identified bases. The skilled person is capable of identifying such erroneously identified bases and knows how to correct for such errors. In case of sequence errors, the sequence of the polypeptides obtainable by expression of the genes present in SEQ ID NO: 1 containing the nucleic acid sequences coding for the polypeptides should prevail.

## Examples

*MATERIAL AND METHODS*

*Preamble : Standard experimental conditions*

All experiments disclosed herein are variations on the standard conditions that are described hereunder. The variations concern the type and concentration of the substrates and the calculation procedures. Experiments were run at least in triplicate and the fluorescence curves were exported to excel sheets for further calculation. All procedures were performed at 37°C.

*Blood and plasma preparation*

Blood (9 volumes) was aseptically drawn in vacutainer tubes (BD Vacutainer System, Roborough, Plymouth, UK) containing 3.2% sodium citrate (1 volume), from the antecubital vein of healthy subjects after obtaining their informed consent according to the Helsinki Declaration. The blood was kept at room temperature ($\pm$ 22 °C) and further processed within 4 hours from withdrawal. Platelet rich plasma (PRP) was obtained by centrifugation of blood at 240g for 15 minutes and platelet poor plasma (PPP) by centrifugation twice at 2630g for 10 min. PPP was aliquoted in 1 ml portions and stored at -80°C for further use.

*Reagents*

BSA5 and BSA60 buffer contain 20 mmol/L HEPES, 140 mmol/L NaCl and 5 or 60 mg/mL bovine serum albumin (BSA; Sigma, St Louis, MO, USA) with a pH of 7.35. The calibrator, $\alpha_2$macroglobulin-thrombin ($\alpha$2M-T) complex, was prepared in-house as described previously in Hemker et al (Pathophysiol Haemost Thromb, 2003; 33: 4-15). Similarly a plasmin-$\alpha$2macroglobulin complex was prepared. Alternatively, for substrates that are too big to react with $\alpha$2macroglobulin-thrombin complex, a stoichiometric complex of prothrombin and staphylocoagulase was used as a calibrator. Recombinant human tissue factor (TF; Innovin®) was from Dade Behring, Marburg, Germany. Recombinant human tissue plasminogen activator (tPA Alteplase) was from Boehringer Ingelheim Phospholipid vesicles (PV) contained 20 mol% phosphatidylserine, 60 mol% phosphatidylcholine and 20 mol% phosphatidyl-ethanolamine (Avanti, Alabaster, AL, USA).

*Standard experiment*

Calibrated automated thrombinography is executed as disclosed in Hemker et al. (Thromb Haemost 2000; 83: 589-91) or Hemker et al. (Pathophysiol Haemost Thromb, 2002; 32: 249-253) or WO2003/093831 A1.

*Plasma in solution:* The TG experiments in the examples are variations on the standard experiment in which thrombin generation in plasma is measured in a 96-wells polystyrene microplate (Thermo Electron Corporation, Milford, MA, USA). All wells contained 80 $\mu$l plasma and 20 $\mu$l of a solution containing 0.5 - 5 pM tissue factor (TF) and 4 $\mu$M phospholipids (PL). To calibrator wells, 20 $\mu$l of calibrator was added instead so as to obtain the equivalent of 100 nM thrombin activity. Thrombin generation (TG) was initiated by the addition of 20 $\mu$l of substrate (standard: Z-Gly-Gly-Arg-AMC, 417 $\mu$M) and CaCl2 (16.6 mM).

*Main steps of the TG assay in solution:*

    **A.** Addition of 80 $\mu$l plasma to each well
    **B.** Addition of 20 $\mu$l TF/phospholipids mix or 20 $\mu$l calibrator to each well
    **C.** Pre-heating of the plate for 10 min at 37°C
    **D.** Addition of 20 $\mu$l FluCa kept at 37°C prior to the addition of the reaction mixture.
    **E.** Immediate kinetic measurement of fluorescence in time (exc 390 nm / em 460 nm)

*Plasma or whole blood in a matrix:*

Surface fluorescence in plasma and in whole blood has been essentially measured in the same way as published by Ninnivagi and Apitz for whole blood (Ninivaggi M, Apitz-Castro R, Dargaud Y, de Laat B, Hemker HC, Lindhout T. Whole blood thrombin generation monitored with a calibrated automated thrombogram-based assay. Clin.Chem. 2012,58:1252-1259) or in WO2006/117246. Paper discs of 5.5 mm diameter and 180 $\mu$m thickness (Whatman 589/1, Whatman GmbH, Dassel, Germany) were placed in flat bottom wells of a 96-wells polystyrene microplate (Thermo Electron Corporation, Milford, MA, USA).

Separately, 30 $\mu$L of citrated whole blood was mixed with 10 $\mu$L (p-tosyl-Gly-Pro-Arg)$_2$-Rho (1.8 mmol/L) and 20 $\mu$L TF and CaCl$_2$ (1.5 pmol/L and 50 mmol/L, respectively). For TG in plasma, 30 $\mu$L PPP was mixed with 10 $\mu$L (p-tosyl-Gly-Pro-Arg)$_2$-Rho (1.8 mmol/L) and 20 $\mu$L trigger solution containing TF (3 pmol/L) and CaCl$_2$ (50 mmol/L). Immediately after mixing, a sample of 5 $\mu$L was pipetted on paper disks and covered with 40 $\mu$L of mineral oil (USB Corporation, Cleveland, OH, USA). The final concentrations were: 50% whole blood or PPP, 0.5 pmol/L TF, 16.7 mmol/L CaCl$_2$, and 300 $\mu$M Rho-based substrate. In a parallel calibration experiment, the TF containing solution was replaced by 20 $\mu$L human thrombin calibrator ($\alpha$2M:T, 300 nmol/L thrombin activity). Fluorescence signal was recorded with a Fluoroskan Ascent microplate fluorometer excitation wavelength 485 nm and emission wavelength 538 nm (Thermolabsystems, Helsinki, Finland).

*Main steps of the TG assay on a matrix*

    **A.** Preparation of paper matrices and placement into a flat-bottom 96 wells plate
    **B.** Addition of the fluorogenic substrate solution to the plasma or to whole blood
    **C.** Pre-heating of the reagents and whole blood for 2 min at 37°C
    **D.** Mixing of the whole blood and the trigger mix or calibrator 1:1
    **E.** Pipetting 5 $\mu$L reaction mix onto the paper matrix
    **F.** Pipetting 40 $\mu$L of mineral oil onto the paper matrix
    **G.** Immediate measurement of fluorescence in time (exc 485 nm / em 538 nm)

*Substrates*

The substrates were commercially obtained or donated by laboratories where they have been synthesized by methods known to the art. As e.g. described in patents WO 00/52199 ((p-tosyl-Gly-Pro-Arg)$_2$-Rho) and FR 2 978 163 (p-tosyl-Gly-Pro-Arg-Rho-Ac).

A particularly suitable substrate is one (Patent Application FR 2 978 164) that contains rhodamine 110 as a fluorophore and in which one of its symmetrical reactive groups is bound to an arginine containing peptide so as to create a site vulnerable to thrombin action and the other reactive group is acetylated. The acetyl chain quenches the fluorescence of rhodamine 110 less than an arginine containing peptide does. This ensures a basic fluorescence of the substrate itself that is advantageous for applying the ratio method.

A third useful substrate is e.g. mPEG-Gly-Pro-Arg-RhoAc where the end group of the peptide is a bulky polyethylene glycol (PEG) with Mr = 400--40000 which increase the hydrodynamic radius of the molecule so as to make it less reactive to thrombin that is bound to $\alpha_2$macroglobulin (Patent Application WO 00/52199).

*Plasmin generation experiments*

*Materials and methods*

Fluorogenic substrates: ButylOxyCarbonyl-Glu-Lys-Lys-AMC (BocGKKAMC)

Buffers: BSA5: 20 mM Hepes, 140 mM NaCl, 5 mg/ml BSA, pH 7.35; BSA60:20 mM Hepes, 60 mg/ml BSA, pH 7.35; $CaCl_2$ 1 M in water.

Stock solutions: SC-FII, prepared in house 1$\mu$M; Plasmin-$\alpha$2macroglobulin complex, prepared in house 1 $\mu$M; rec Human recombinant tissue factor (TF) (Innovin; Siemens) 6$\mu$M. Phospholipids (PS:PC:PE, 2:6:2) prepared in house 1 mM, Human recombinant tissue plasminogen activator (tPA), (Alteplase Boehringer Ingelheim) 36 kIU, Final concentrations used: SC-FII, 100 nM, Plasmin-$\alpha$2macroglobulin 100 nM: tPA 300 IU. Substrate 300 $\mu$M,

Execution of the experiment:

To 40 $\mu$L of plasma was added 20 $\mu$L of the substrate solution (1,35 mM in BSA 60 buffer) so as to obtain a concentration of 450 $\mu$M.

To 40 $\mu$L of this mixture were added 20 $\mu$L of the activation mixture containing the indicated concentration of $Ca^{++}$, TF, PI, tPA in BSA 5 buffer. 5 $\mu$L of this of this solution was transferred to three 6 mm diameter paper rounds at the (flat) bottom of a 96-well plate. 40 $\mu$L of liquid paraffin was added to all the wells to prevent evaporation. The plate was read in a Fluoroscan Ascent fluorometer at 37°C. Samples were measured every 10s at 390-460 nm for 50 minutes.

**Example 1**

*Decrease of experimental scatter through use of ratio determination*

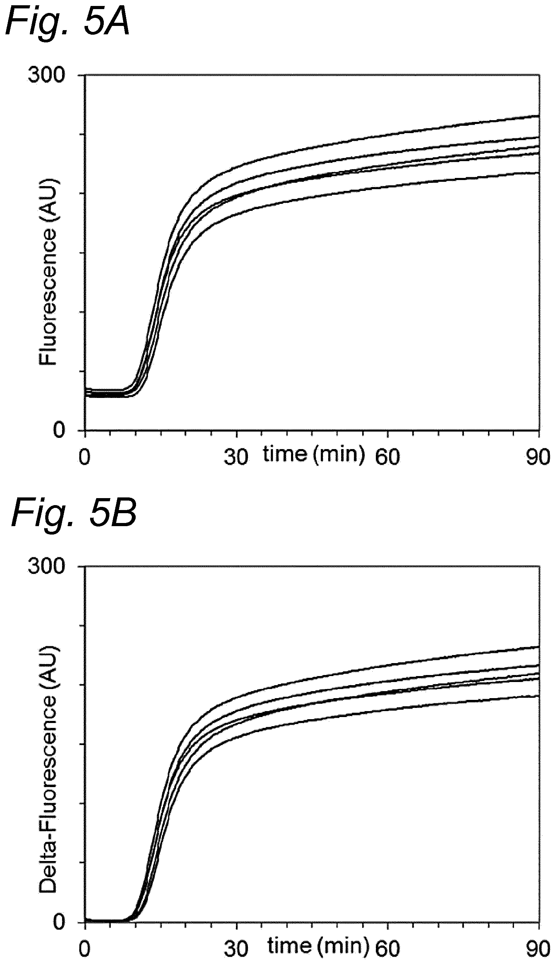

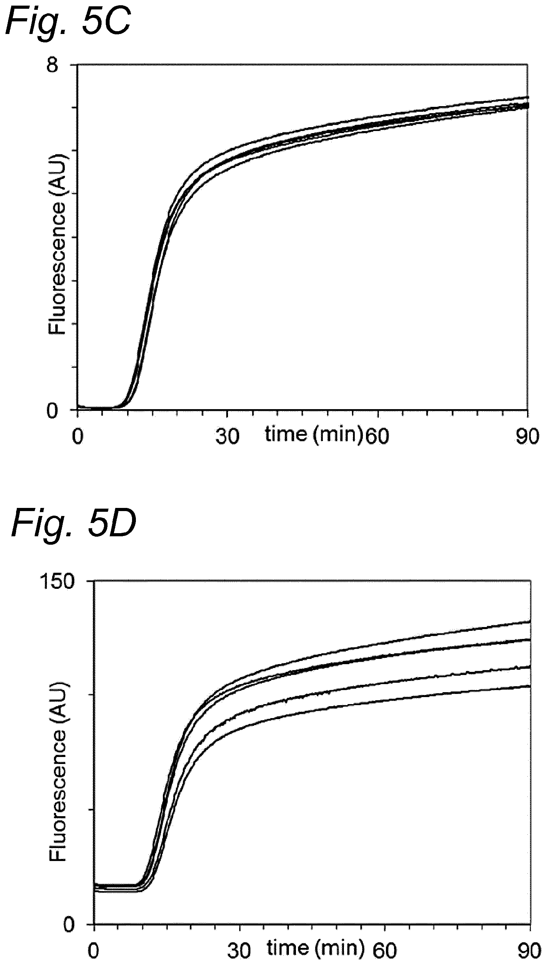

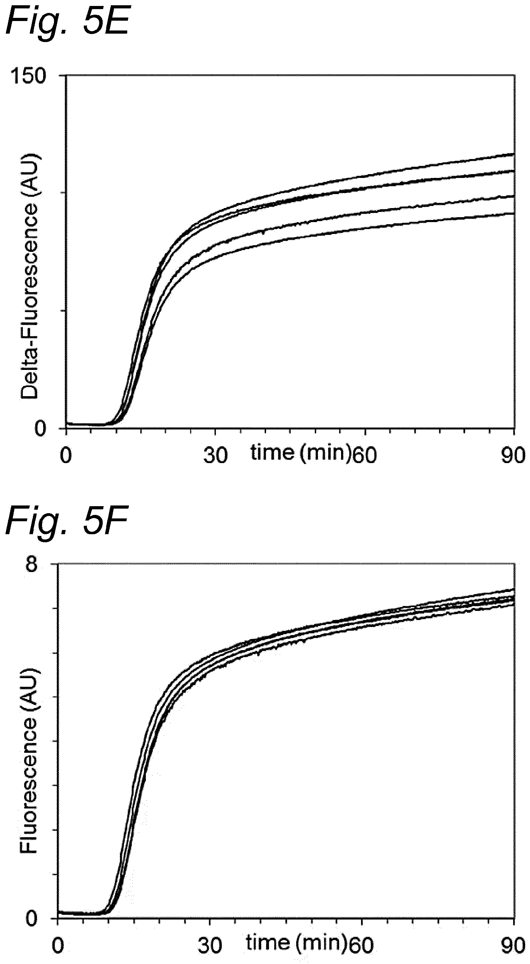

Fig. 5 shows a standard thrombin generation experiment with substrate mPEG-Gly-Pro-Arg-RhoAc in paper (frames A and D) and the effect of subtracting the blank (frames B and E) versus dividing by the blank (frame C and F). It is seen at both 300 and 75 $\mu$M concentration of substrate the experimental scatter of the raw data is considerably decreased after application of the ratio calculation.

**Example 2**

*The conversion of the normalized ratio curve into a thrombin activity curve.*

A standard thrombin generation experiment with 5 pM tissue factor was carried out, using Z-Gly-Gly-Arg-AMC as a substrate at the concentrations 108- and 416 b$\mu$M with 1$\mu$M of AMC added. Fom the fluorescence data the normalized ratio was calculated as $R_t=(F_{exp.t}-F_0)/(F_{max}-F_0)$ (dashed lines). From this line the first derivatives were calculated (dotted lines) and the thrombin concentrations were calculated as $E_t=dR_t/d_t.(S_0+K_m/(1-R_t))/k_{cat}$ with $K_m$=900 $\mu$M, $k_{cat}$=1.9 s$^{-1}$ and $S_0$= 104 $\mu$M (Fig. 6 frame A) and 416 $\mu$M (Fig. 6 frame B).

**Example 3**

*An experiment using mPEG-Gly-Gly-Arg-Rho-Ac.*

In 1:2 diluted normal plasma adsorbed on filter paper a concentration series of mPEG-Gly-Pro-Arg-RhoAc (75, 150, 225, 300, 450 and 600 $\mu$M) was converted by 100 nM of staphylocoagulase-thrombin and the resulting fluorescence recorded as further described under materials and methods. (Fig. 7 frame A)
$F_{max}$ is determined as the intercept with the abscissa of a plot of the first derivative of these fluorescence values against the fluorescence values themselves. It appeared that both $F_0$ and $F_{max}$ were proportional to $S_0$. The fact that the normalized ratio, calculated as $R_t=(F_{exp.t}-F_0)/(F_{max}-F_0)$ is independent of $S_0$ (frame B) means that $F_{expt.t}$, is proportional to $S_0$ as well. This means that the reaction velocity is proportional to the substrate concentration. According to the Michaelis-Menten relation ($dP/dt=k_{cat}.E.S/(K_m+S)$), it follows that $K_m>>S$ (i.e. $K_m>>600$ μM), so that the relation $E_t=dR_t/dt.(S_0+K_m/(1-R_t))/(k_{cat})$ reduces to $E_t=dR_t/dt.(K_m/k_{cat})/(1-R_t)$. Indeed the plot of $dR_t/dt$ against $R_t$ (Fig. 7C) is a straight line (Aside: The non-linear start of the curve is caused by a temperature effect and is neglected) that cuts the abscissa at $R_t=1$ and the ordinate at $E.k_{cat}/K_m$. For the latter intercept a value of 0.0088 is found. The enzyme concentration is 100 nM so that $K_m/k_{cat}=11,36$ μM.min and $E_t=11364$ $dR_t/dt./(1-R_t)$ at any substrate concentration $\leq 600$ μM. Because the variations between similar experiments are eliminated through use of the normalized ration this formula can be applied to all experiments carried out under similar conditions and provided to the user, thus eliminating the need for running separate calibration experiments.

## Example 4

*Thrombin generation in normal plasma in filter paper with a large MW substrate.*

A thrombin generation experiment was carried out on filter paper using normal plasma triggered with 5 pM tissue factor as described in the materials and methods section, using mPEG-Gly-Pro-Arg-RhoAc as a substrate. This high molecular weight substrate is particularly useful because it is hardly sensitive to the action of macroglobulin-thrombin, so that only free thrombin is measured. Because $S_0 << K_m$ (see above) it does not compete with antithrombins and therefore does not retard thrombin breakdown. This unexpected property makes it excellently suited for the application of the ratio method.

A standard thrombin generation experiment in paper with this substrate at six concentrations is shown in Fig. 8.

The normalized ratios are calculated as in the previous experiment. The ratios obtained at several substrate concentrations are found to superimpose (Fig. 8B), confirming that the substrate concentrations are much lower than $K_m$.

The Fig. 8C shows the thrombin concentrations calculated from the date in Fig. 8B by using the formula $E_t=11364(1-R_t).dR_t/dt$ as derived in the previous example. Fig. 8C shows that the values found for the area under the thrombin generation curve (endogenous thrombin potential, ETP) and the peak are independent of the substrate concentration used within the limits of experimental error.

## Example 5.

*A plasmin generation experiment*

A plasmin generation experiment was carried out as described in the methods section with Boc-Glu-Lys-Lys-AMC (300 μM) as a substrate to which 8 μM AMC was added. The following combinations of tissue factor (TF) and tissue plasminogen activator (tPA) were tested: a: 0 TF, 0 tPA; b:1pm TF,0 tPA; c: 1 pM TF,200 PM tPA; d: 0 TF,200 tPA. No plasmin was seen when no tPA was added, confirming the specificity of the substrate. These curves were omitted. We simultaneously recorded a fluorescence curve from plasmin-α2-macroglobulin complex, equivalent to 100 nM plasmin. From this curve, run to completion we found $F_{max}$ to be 1778 AU, whereas $F_0$ as measured during the lag-times, was 49,2 AUs.

In Fig. 9A the course of the normalized ratios is shown and in Fig. 9B their first derivatives.

It appeared again that a plot **of dRt/dt** against $R_t$ was a straight line (like in example 3) so that again the formula $E_t=dR_t/dt.(S_0+K_m/(1-R_t))/k_{cat}$ we calculated the course of the concentration of plasmin activity from these sets of data (Fig. 9C). The steady activity found for the calibrator experiment (Fig. 9C), confirms the validity of the calculation procedure.

We observe a large and steadily increasing plasmin activity that must be attributed to an ongoing plasmin generation together with the formation of α2-macroglobulin-plasmin complex. When these are subtracted, using methods analogous to those known to the art for subtracting α2M-thrombin activity in thrombin generation experiments, then the peaked curves in the right frame result, which thus show an initial burst of plasmin.

The plot of the first derivative of fluorescence data against fluorescence is a straight line (Fig. 13, right frame) hence the plot of the first derivative of the normalized ratio against the normalized ratio is also a straight line. This shows that $S_0 << K_m$ so that
Again the formula $E_t=dR_t/dt/(1-R_t).(K_m/k_{cat})$ applies. From the plot of $dR_t/dt$ against $R_t$ at constant E (100 nM) we read the value the of $E.k_{cat}/K_m$ as the intercept with the ordinate and hence we know $K_m/k_{cat}$, which we found to be 8660 nM.min.

Like in the previous example we compare the ratio- and the standard method and find them to be similar within the limits of experimental error (Fig. 9D). In practice it cannot always be expected that these curves superimpose for all substrates used because it is not to be expected that α2M-plasmin and plasmin itself always share the same kinetic constants and it may be necessary to slightly change the values to obtain superposition.

## Example 6

*A whole blood experiment*

Whole blood thrombin generation can be especially useful in point of care instruments because no centrifugation is required. In such instruments, under point of care conditions, it is not well possible to run a calibration experiment in parallel with the test. The ratio method thus is perfectly suited to serve under these circumstances.

We carried out a whole blood experiment, as described under methods and we also determined the fluorescence caused by a fixed amount of α2macroglobulin-thrombin under the same conditions, in order to compare the old way of calculation to the method disclosed here. The experiments are carried out in a filter paper as described under material and methods using 100- and 200 µM of Z-Gly-Gly-Arg-Rhod-Ac as a substrate.

In the traditional method the fluorescence data from the calibrator curves should be first subjected to a mathematical procedure that compensates for substrate consumption, i.e. renders the calibrator curve straight. (Any procedure that achieves this effect will do, here we use $F_{corrected}=-A.Ln(1-F_{measured}/A)$, where A is a constant that is adjusted so as to have the desired straightening effect.) This same procedure is then applied to the experimental TG curves. This is shown in Fig. 10A; the dotted curves are the observed fluorescence values. The drawn lines are the corrected values. The first derivatives of these curves are shown in Fig. 10B. The $K_m$ and $k_{cat}$ values of the horizontal lines represent the concentration of thrombin in the calibrator, i.e. provide the calibration factor by which the curved lines should be multiplied to yield thrombin generation curves. These curves are shown in Fig. 10C.

In the ratio method we first determine the normalized ratio $R_t$ (Fig. 11A) as explained above. Then we determine $K_m$ and $k_{cat}$ and we calculate the thrombin concentration from $E_t=dR_t/dt.(K_m/k_{cat}).(1-R_t)^{-1}$ (Fig. 11B) using the kinetic constants provided from experiments as shown in example 3. In Fig. 11A&B also an experiment with 100 nM stable thrombin activity is shown, not because it is required as a calibrator but in order to illustrate that the procedure followed indeed in the end renders a horizontal line, i.e. a stable value at the correct thrombin concentration. It thus is a check that the correct values for $k_{cat}$ and $K_m$ have been used but not required for the calculation *per se.* Fig. 11C compares the results of the standard- and the ratio method.

## Example 7

*A practical shortcut for determining ρ in non-macro substrates.*

As explained above, ρ, the ratio of the initial fluorescence to the fluorescence at complete substrate conversion, which is required to calculate the normalized ratio, is a specific property of the molecule of a substrate. Due to contamination of the substrate with product, either willfully or accidentally through decomposition of the substrate during storage, this value may become lower than its theoretical optimum. It is not absolutely necessary to re-determine this value in the manner as explained previously. Any thrombin generation curve will, after thrombin generation is over and no free thrombin is left, maintain a certain A2M-thrombin activity that will continue to convert substrate, the "tail". (The exceptions are macrosubstrates that are not converted by α2M-thrombin). In the end α2M-thrombin activity will tend to full conversion of the substrate, independent of the amount of enzymatic activity. It therefore is possible to use the part of a TG curve to determine the maximal fluorescence that in the end will be attained. In Fig. 12A two experimental fluorescence traces from TG experiments are shown. The part of these traces that corresponds to the "tail" are rendered as bold lines. Of these lines the ratios - so not the normalized ratios - are obtained by dividing the experimental values by the start value during the lag time (=$F_0$). In Fig. 12B the first derivatives of these ratio curves is shown. Then the first derivatives are plotted against the corresponding ratios, i.e. the experimental fluorescence values divided by $F_0$ (Fig. 12C).

When the part corresponding to the "tail" is extrapolated to the abscissa, the maximal attainable ratio, i.e. $R_{max}$, is found.

## REFERENCES

1. Hemker et al; Continuous registration of thrombin generation in plasma, its use for the determination of the thrombin potential, Thromb.Haemost 1993, 70:617-624
2. Hemker et al; Thromb Haemost 2000; 83: 589-91
3. Hemker et al; Pathophysiol Haemost Thromb, 2003; 33: 4-15

4. Wagenvoord et al; The paradoxical stimulation by a reversible thrombin inhibitor of thrombin generation in plasma measured with thrombinography is caused by alpha-macroglobulin-thrombin. Journal of thrombosis and haemostasis : 2010, 8:1281-9

5. Hemker et al; Thromb Haemost 2000; 83: 589-91

6. Hemker et al; Pathophysiol Haemost Thromb, 2002; 32: 249-253

7. Ninivaggi et al; Whole blood thrombin generaton monitored with a calibrated automated thrombogram-based assay. Clin.Chem. 2012,58:1252-1259

## Claims

1. Method for *in vitro* determination of the activity over time of at least one proteolytic enzyme in a biological sample, preferably a plasma sample or a whole blood sample, comprising determining over time the conversion of a fluorogenic substrate specific for said proteolytic enzyme, said method comprising the steps of:

   a) contacting the sample with at least one fluorogenic substrate for a proteolytic enzyme wherein said fluorogenic substrate has native fluorescence and measuring the native fluorescence $F_0$, and contacting said sample with an agent triggering the generation of said proteolytic enzyme within the sample;
   b) generating a fluorescent signal over time curve by measuring, over time while said proteolytic enzyme is generated within the sample, the time course of at least one experimental fluorescence signal $F_{exp}.t$ emitted from the sample as a result of the release of a fluorescent group of the fluorogenic substrate specific for said proteolytic enzyme upon enzymatic action of the generated proteolytic enzyme on said fluorogenic substrate;
   c) measuring in the same or in a similar sample, $F_{max}$ the maximal fluorescence that is obtained when all substrate has been converted into fluorescent product;
   d) generating a normalized ratio over time curve by converting the values of $F_{exp.t}$ measured in step b) to values defined as the normalized ratio values $R_t$ according to the equation $R_t = (F_{exp.t}-F_0)/(F_{max}-F_0)$, using $F_0$ and $F_{max}$ as obtained in steps a, b and c;
   e) generating a proteolytic enzyme generation over time curve by applying the formula $E_t=dR_t/dt.(S_0+K_m/(1-R_t))/(k_{cat})$, where $S_0$ is the initial substrate concentration and $K_m$ and $k_{cat}$ are kinetic constants that govern the interaction between the proteolytic enzyme and the fluorogenic substrate.

2. Method for *in vitro* determination of the activity over time of at least one proteolytic enzyme in a biological sample, preferably a plasma sample or a whole blood sample, comprising determining over time the conversion of a fluorogenic substrate specific for said proteolytic enzyme, said method comprising the steps of:

   a) contacting the sample with at least one fluorogenic substrate for a proteolytic enzyme wherein said fluorogenic substrate has native fluorescence and measuring the native fluorescence $F_0$, and contacting said sample with an agent triggering the generation of said proteolytic enzyme within the sample;
   b) generating a fluorescent signal over time curve by measuring, over time while said proteolytic enzyme is generated within the sample, the time course of at least one experimental fluorescence signal $F_{exp}.t$ emitted from the sample as a result of the release of a fluorescent group of the fluorogenic substrate specific for said proteolytic enzyme upon enzymatic action of the generated proteolytic enzyme on said fluorogenic substrate;
   c) calculating $F_{max}$ from the experimentally obtained $F_0$ and the constant $M=F_{max}/F_0$ obtainable or obtained from a reference experiment;
   d) generating a normalized ratio over time curve by converting the values of $F_{exp.t}$ measured in step b) to values defined as the normalized ratio values $R_t$ according to the equation $R_t = (F_{exp.t}-F_0)/(F_{max}-F_0)$, using $F_0$ and $F_{max}$ as obtained in steps a, b and c;
   e) generating a proteolytic enzyme generation over time curve by applying the formula $E_t=dR_t/dt.(S_0+K_m/(1-R_t))/(k_{cat})$, where $S_0$ is the initial substrate concentration and $K_m$ and $k_{cat}$ are kinetic constants that govern the interaction between the proteolytic enzyme and the fluorogenic substrate.

3. The method according to claim 1 or 2, wherein $F_0$ is the fluorescence value during the lag time of enzyme generation and/or wherein the proteolytic enzyme, is thrombin and/or plasmin.

4. The method according to claim 1, 2 or 3, wherein the fluorogenic substrate further comprises a fixed amount of fluorescent product that is between 0.1% and 10 % of the fluorogenic substrate concentration in the sample.

5. The method according to any one of claims 1 to 4, wherein the fluorogenic substrate is a rhodamine-based fluorogenic substrate, preferably a rhodamine-based peptide, preferably encompassing a Rhodamine110 fluorophore, or is an AMC-based fluorogenic substrate, wherein, optionally, the sample is supplemented with an amount of the fluorescent product corresponding to the fluorogenic substrates or a different fluorescent product, equal to between 0.1% and 10% of the fluorogenic substrate in the sample; and/or,

wherein the fluorogenic substrate comprises at least one oligopeptide having a sequence of 1 to 30 amino acid residues coupled to a fluorescent molecule, wherein the oligopeptide preferably has a terminal lysine or terminal arginine coupled to the fluorescent molecule.

**6.** A method according to claim 5, wherein the fluorogenic substrate is Rhodamine 110-based monopeptide, wherein one of the symmetrical reactive NH-groups in Rhodamine 110 is bound to the oligopeptide at a terminal lysine or arginine residue and the other reactive NH-group is acetylated; and/or,

wherein the oligopeptide within the substrate is chemically coupled to a hydrophilic group having a molecular weight of at least 5 kD, such as a polyethylene glycol (PEG) group; and/or,

wherein the oligopeptide of the substrate has the amino-acid sequence: Gly-Gly-Arg, Gly-Pro-Arg, Ile-Pro-Arg, Phe-Pipecolic acid (PIP)-Arg, Val-Leu-Lys, Gly-Pro-Lys or Ala-Phe-Lys.

**7.** The method according to any one of claims 1 to 6, wherein the proteolytic enzyme is thrombin and/or plasmin and wherein agent triggering thrombin and/or plasmin generation is selected from the group consisting of: calcium ions, phospholipids, tissue factor (TF), soluble tissue factor (sTF), thromboplastin, kaolin, elagic acid, plasminogen activator, in particular tissue plasminogen activator (tPA), urokinase and streptokinase; and/or,

wherein the biological sample is a citrated blood sample, preferably a citrated and/or platelet-free plasma sample, a platelet poor plasma (PPP) sample or a platelet rich plasma (PRP) sample; and/or, wherein, for the assay, the biological sample is deposited in a container or on a support, such as a chip, preferably a microfluidic chip, said container or support having the fluorogenic substrate adsorbed on a solid opaque matrix placed within the container or adsorbed on the support.

**8.** The method according to any one of claims 1 to 7, wherein the thrombin and plasmin generation over time curves are simultaneously generated, either using a fluorogenic substrate that is sensitive to both enzymes or using a mixture of a fluorogenic plasmin substrate and a fluorogenic thrombin substrate that yield the same fluorescent product.

**9.** Method for *in vitro* detecting or monitoring a haemostatic disease or a thrombotic disease, or for monitoring the treatment of a haemostatic disease or a thrombotic disease, by measuring the effect on thrombin and/or plasmin generation of the administration of haemostatic, antithrombotic or pro- or anti-thrombolytic drugs or agents, by performing a method according to any one of claims 1 to 8.

**10.** Method for *in vitro* detecting or monitoring the interaction of a candidate substance on thrombin and/or plasmin activity in a biological sample, especially a whole blood sample, by performing a method according to any one of claims 1 to 9, wherein said candidate substance is added to the sample to be assayed or is added to the sample during thrombin and/or plasmin generation, preferably for monitoring interaction of coagulation factors or drugs or anticoagulant factors or drugs, more preferably thrombin and/or plasmin inhibitors or plasmin activators, more preferably for screening substances to determine their interacting capacity with thrombin and/or plasmin generation.

**11.** The method according to any one of claims 1 to 10 for *in vitro* measurement of thrombin- or plasmin generating capacity in the biological sample by producing the thrombin- or plasmin generation curve and assessing the area under the curve (Endogenous Thrombin Potential (ETP) or Endogenous Plasmin Potential), the maximal value of the curve (peak) and/or measurement of time to peak of thrombin and/or plasmin.

**12.** A method of *in vitro* determining the activity over time of plasmin in a biological sample obtained from an individual, comprising the steps of:

i) performing the method according to any one of claims 1 to 11 of *in vitro* determination of the activity over time of thrombin in said biological sample, wherein only thrombin generation is triggered, and

ii) performing the method according to any one of claims 1 to 11 of *in vitro* determination of the activity over time of thrombin and plasmin in the biological sample analyzed in step i), wherein both thrombin and plasmin generation are triggered, and

iii) subtracting the output obtained in i) from the output obtained in ii) to obtain a representation of the activity over time of plasmin for the assayed sample.

**Patentansprüche**

1. Verfahren zur In-vitro-Bestimmung der Aktivität von mindestens einem proteolytischen Enzym in einer biologischen Probe, vorzugsweise einer Plasmaprobe oder einer Vollblutprobe, über einen längeren Zeitraum, umfassend das Bestimmen des zeitlichen Verlaufs der Umwandlung eines fluorogenen Substrats, welches für das proteolytische Enzym spezifisch ist, wobei das Verfahren die folgenden Schritte umfasst:

   a) Inkontaktbringen der Probe mit mindestens einem fluorogenen Substrat für ein proteolytisches Enzym, wobei das fluorogene Substrat eine native Fluoreszenz aufweist, und Messen der nativen Fluoreszenz $F_0$, sowie Inkontaktbringen der Probe mit einem Mittel, welches die Bildung des proteolytischen Enzyms in der Probe induziert;

   b) Erstellen einer Fluoreszenzsignalkurve über die Zeit durch Messen über einen längeren Zeitraum, während das proteolytische Enzym in der Probe gebildet wird, des zeitlichen Verlaufs von mindestens einem experimentellen Fluoreszenzsignal $F_{exp}.t$, welches von der Probe als Ergebnis der Freisetzung einer fluoreszierenden Gruppe des fluorogenen Substrats, welches für das proteolytische Enzym spezifisch ist, nach der enzymatischen Einwirkung des gebildeten proteolytischen Enzyms auf das fluorogene Substrat emittiert wird;

   c) Messen in derselben oder in einer vergleichbaren Probe des Wertes $F_{max}$ der maximalen Fluoreszenz, die erhalten wird, wenn das gesamte Substrat in ein fluoreszierendes Produkt umgewandelt wurde;

   d) Erstellen einer Kurve für das normalisierte Verhältnis über die Zeit durch Umrechnung der in Schritt b) gemessenen Werte für $F_{exp}.t$ in Werte, welche definiert sind als normalisierte Verhältniswerte $R_t$, entsprechend der Gleichung $R_t = (F_{exp.t}-F_0)/(F_{max} - F_0)$, unter Verwendung von $F_0$ und $F_{max}$, welche Werte in den Schritten a, b und c erhalten wurden;

   e) Erstellen einer Kurve für die Bildung des proteolytischen Enzyms über die Zeit durch Verwendung der Formel $E_t = dR_t/dt.(S_0+K_m/(1-R_t))/(kcat)$, worin $S_0$ die anfängliche Substratkonzentration ist, und $K_m$ und $k_{cat}$ den kinetischen Konstanten, welche die Interaktion zwischen dem proteolytischen Enzym und dem fluorogenen Substrat regulieren, entsprechen.

2. Verfahren zur In-vitro-Bestimmung der Aktivität von mindestens einem proteolytischen Enzym in einer biologischen Probe, vorzugsweise einer Plasmaprobe oder einer Vollblutprobe, über einen längeren Zeitraum, umfassend das Bestimmen des zeitlichen Verlaufs der Umwandlung eines fluorogenen Substrats, welches für das proteolytische Enzym spezifisch ist, wobei das Verfahren die folgenden Schritte umfasst:

   a) Inkontaktbringen der Probe mit mindestens einem fluorogenen Substrat für ein proteolytisches Enzym, wobei das fluorogene Substrat eine native Fluoreszenz aufweist, und Messen der nativen Fluoreszenz $F_0$, sowie Inkontaktbringen der Probe mit einem Mittel, welches die Bildung des proteolytischen Enzyms in der Probe induziert;

   b) Erstellen einer Fluoreszenzsignalkurve über die Zeit durch Messen über einen längeren Zeitraum, während das proteolytische Enzym in der Probe gebildet wird, des zeitlichen Verlaufs von mindestens einem experimentellen Fluoreszenzsignal $F_{exp}.t$, welches von der Probe als Ergebnis der Freisetzung einer fluoreszierenden Gruppe des fluorogenen Substrats, welches für das proteolytische Enzym spezifisch ist, nach der enzymatischen Einwirkung des gebildeten proteolytischen Enzyms auf das fluorogene Substrat emittiert wird;

   c) Berechnen von $F_{max}$ aus dem experimentell erhaltenen Wert für $F_0$ und der verfügbaren oder aus einem Referenzversuch erhaltenen Konstante $M = F_{max}/F_0$;

   d) Erstellen einer Kurve für das normalisierte Verhältnis über die Zeit durch Umrechnung der in Schritt b) gemessenen Werte für $F_{exp}.t$ in Werte, welche definiert sind als normalisierte Verhältniswerte $R_t$, entsprechend der Gleichung $R_t = (F_{exp.t} - F_0)/(F_{max} - Fo)$, unter Verwendung von $F_0$ und $F_{max}$, welche Werte in den Schritten a, b und c erhalten wurden;

   e) Erstellen einer Kurve für die Bildung des proteolytischen Enzyms über die Zeit durch Verwendung der Formel $E_t = dR_t/dt.(S_0+K_m/(1-R_t))/(kcat)$, worin $S_0$ die anfängliche Substratkonzentration ist, und $K_m$ und $k_{cat}$ den kinetischen Konstanten, welche die Interaktion zwischen dem proteolytischen Enzym und dem fluorogenen Substrat regulieren, entsprechen.

3. Verfahren nach Anspruch 1 oder 2, wobei $F_0$ dem Fluoreszenzwert während der Latenzzeit bis zur Bildung des Enzyms entspricht und/oder wobei das proteolytische Enzym Thrombin und/oder Plasmin ist.

4. Verfahren nach Anspruch 1, 2 oder 3, wobei das fluorogene Substrat weiterhin eine festgelegte Menge eines fluoreszierenden Produkts, welche zwischen 0,1% und 10% der Konzentration des fluorogenen Substrats in der Probe beträgt, umfasst.

**5.** Verfahren nach einem der Ansprüche 1 bis 4, wobei das fluorogene Substrat ein von Rhodamin abgeleitetes fluorogenes Substrat, vorzugsweise ein von Rhodamin abgeleitetes Peptid, das vorzugsweise ein Rhodamin 110-Fluorophor umfasst, oder ein von AMC abgeleitetes fluorogenes Substrat ist, wobei gegebenenfalls die Probe mit einer Menge des fluoreszierenden Produkts, welches den fluorogenen Substraten entspricht, oder eines anderen fluoreszierenden Produkts, wobei die Menge einer Menge zwischen 0,1% und 10% der Konzentration des fluorogenen Substrats in der Probe entspricht, angereichert wird; und/oder
wobei das fluorogene Substrat mindestens ein Oligopeptid mit einer Sequenz bestehend aus 1 bis 30 Aminosäureresten umfasst, welches an ein fluoreszierendes Molekül gekoppelt ist, wobei das Oligopeptid vorzugsweise einen terminalen Lysinrest oder einen terminalen Argininrest aufweist, der mit dem fluoreszierenden Molekül verbunden ist.

**6.** Verfahren nach Anspruch 5, wobei das fluorogene Substrat ein von Rhodamin 110 abgeleitetes Monopeptid ist, worin eine der symmetrischen reaktiven NH-Gruppen in dem Rhodamin 110-Molekül über einen terminalen Lysin- oder Argininrest an das Oligopeptid gebunden ist und die andere reaktive NH-Gruppe acetyliert ist; und/oder wobei das Oligopeptid innerhalb des Substrats durch chemische Kopplung an eine hydrophile Gruppe mit einem Molekulargewicht von mindestens 5 kD, wie eine Polyethylenglykol (PEG)-Gruppe, gebunden ist; und/oder
wobei das Oligopeptid des Substrats die folgende Aminosäuresequenz aufweist: Gly-Gly-Arg, Gly-Pro-Arg, Ile-Pro-Arg, Phe-Pipecolinsäure (PIP)-Arg, Val-Leu-Lys, Gly-Pro-Lys oder Ala-Phe-Lys.

**7.** Verfahren nach einem der Ansprüche 1 bis 6, wobei das proteolytische Enzym Thrombin und/oder Plasmin ist und wobei das Mittel, welches die Bildung von Thrombin und/oder Plasmin induziert, gewählt ist aus der Gruppe, bestehend aus: Calciumionen, Phospholipiden, Gewebefaktor (TF), löslichem Gewebefaktor (sTF), Thromboplastin, Kaolin, Ellagsäure, Plasminogen-Aktivator, insbesondere Gewebeplasminogen-Aktivator (tPA), Urokinase und Streptokinase; und/oder
wobei die biologische Probe eine Citratblutprobe, vorzugsweise eine mit Citrat versetzte und/oder Thrombozyten-freie Plasmaprobe, eine Thrombozyten-arme Plasmaprobe (PPP) oder eine Thrombozyten-reiche Plasmaprobe (PRP) ist; und/oder
wobei für den Assay die biologische Probe in einen Behälter eingebracht wird oder auf einen Träger, wie einen Chip, vorzugsweise einen Mikrofluid-Chip, aufgebracht wird, wobei der Behälter oder der Träger das fluorogene Substrat adsorbiert an eine feste undurchlässige Matrix, welche in dem Behälter angeordnet ist oder an den Träger adsorbiert ist,
umfassen.

**8.** Verfahren nach einem der Ansprüche 1 bis 7, wobei die Kurven für die Bildung von Thrombin und Plasmin über die Zeit gleichzeitig erstellt werden, entweder unter Verwendung eines fluorogenen Substrats, welches gegenüber beiden Enzymen empfindlich ist, oder unter Verwendung einer Mischung aus einem fluorogenen Substrat für Plasmin und einem fluorogenen Substrat für Thrombin, welche Substrate das gleiche fluoreszierende Produkt ergeben.

**9.** Verfahren zum Nachweis oder zur Überwachung einer hämostatischen Erkrankung oder einer thrombotischen Erkrankung oder zur Überwachung der Behandlung einer hämostatischen Erkrankung oder einer thrombotischen Erkrankung *in vitro* durch Messen der Auswirkung der Verabreichung von hämostatischen, antithrombotischen oder pro- oder antithrombolytischen Wirkstoffen oder Mitteln auf die Bildung von Thrombin und/oder Plasmin, indem ein Verfahren nach einem der Ansprüche 1 bis 8 durchgeführt wird.

**10.** Verfahren zum Nachweis oder zur Überwachung *in vitro* der Interaktion einer Kandidatensubstanz mit der Aktivität von Thrombin und/oder Plasmin in einer biologischen Probe, insbesondere einer Vollblutprobe, indem ein Verfahren nach einem der Ansprüche 1 bis 9 durchgeführt wird, wobei die Kandidatensubstanz zu der zu untersuchenden Probe zugegeben wird oder während der Bildung von Thrombin und/oder Plasmin zu der Probe zugegeben wird, vorzugsweise zur Überwachung der Interaktion von gerinnungsfördernden Faktoren oder Arzneimitteln oder gerinnungshemmenden Faktoren oder Arzneimitteln, mehr bevorzugt von Thrombin- und/oder Plasmin-Inhibitoren oder Plasmin-Aktivatoren, insbesondere für das Screening von Substanzen, um deren Interaktionsfähigkeit mit der Bildung von Thrombin und/oder Plasmin zu bestimmen.

**11.** Verfahren nach einem der Ansprüche 1 bis 10 zur In-vitro-Messung der Thrombin- und/oder Plasmin-Bildungskapazität in der biologischen Probe durch Erstellen einer Kurve für die Bildung von Thrombin und/oder Plasmin und das Bestimmen der Fläche unter der Kurve (Endogenes Thrombin-Potenzial (ETP) oder Endogenes Plasmin-Potenzial) sowie des maximalen Werts der Kurve (Peak) und/oder das Messen der Zeitdauer bis zum Erreichen des Peaks von Thrombin und/oder Plasmin.

12. Verfahren zur In-vitro-Bestimmung der Aktivität von Plasmin über die Zeit in einer biologischen Probe, welche von einem Individuum erhalten wurde, umfassend die folgenden Schritte:

i) Durchführen des Verfahrens nach einem der Ansprüche 1 bis 11 einer In-vitro-Bestimmung der Aktivität von Thrombin über die Zeit in der biologischen Probe, wobei nur die Bildung von Thrombin induziert wird, und
ii) Durchführen des Verfahrens nach einem der Ansprüche 1 bis 11 einer In-vitro-Bestimmung der Aktivität von Thrombin und Plasmin über die Zeit in der in Schritt i) analysierten biologischen Probe, wobei die Bildung von sowohl Thrombin als auch von Plasmin induziert wird, und
iii) Subtrahieren des in i) erhaltenen Ergebnisses von dem in ii) erhaltenen Ergebnis, um eine Darstellung der Aktivität von Plasmin über die Zeit für die untersuchte Probe zu erhalten.

**Revendications**

1. Procédé de détermination *in vitro* de l'activité dans le temps d'au moins une enzyme protéolytique dans un échantillon biologique, de préférence un échantillon de plasma ou un échantillon de sang total, comprenant la détermination dans le temps de la conversion d'un substrat fluorogène spécifique à ladite enzyme protéolytique, ledit procédé comprenant les étapes consistant à :

a) mettre en contact l'échantillon avec au moins un substrat fluorogène pour une enzyme protéolytique dans lequel ledit substrat fluorogène a une fluorescence native et mesurer la fluorescence native $F_0$, et mettre en contact ledit échantillon avec un agent déclenchant la génération de ladite enzyme protéolytique dans l'échantillon ;
b) générer une courbe de signal fluorescent dans le temps en mesurant, dans le temps pendant que ladite enzyme protéolytique est générée dans l'échantillon, l'évolution dans le temps d'au moins un signal de fluorescence expérimental $F_{exp}.t$ émis par l'échantillon en résultat de la libération d'un groupe fluorescent du substrat fluorogène spécifique à ladite enzyme protéolytique lors de l'action enzymatique de l'enzyme protéolytique générée sur ledit substrat fluorogène ;
c) mesurer dans le même échantillon ou dans un échantillon similaire, la fluorescence maximum $F_{max}$ qui est obtenue lorsque tout le substrat a été converti en produit fluorescent ;
d) générer une courbe de rapport normalisé dans le temps en convertissant les valeurs de $F_{exp.t}$ mesurées à l'étape b) en valeurs définies comme les valeurs de rapport normalisé $R_t$ selon l'équation $R_t = (F_{exp.t}-F_0)/(F_{max}-F_0)$, en utilisant $F_0$ et $F_{max}$ obtenues aux étapes a, b et c ;
e) générer une courbe de génération d'enzyme protéolytique dans le temps en appliquant la formule $E_t = dR_t/dt.(S_0+K_m/(1-R_t))/(k_{cat})$, où $S_0$ est la concentration initiale de substrat et $K_m$ et $k_{cat}$ sont des constantes cinétiques qui régissent l'interaction entre l'enzyme protéolytique et le substrat fluorogène.

2. Procédé de détermination *in vitro* de l'activité dans le temps d'au moins une enzyme protéolytique dans un échantillon biologique, de préférence un échantillon de plasma ou un échantillon de sang total, comprenant la détermination dans le temps de la conversion d'un substrat fluorogène spécifique à ladite enzyme protéolytique, ledit procédé comprenant les étapes consistant à :

a) mettre en contact l'échantillon avec au moins un substrat fluorogène pour une enzyme protéolytique dans lequel ledit substrat fluorogène a une fluorescence native et mesurer la fluorescence native $F_0$, et mettre en contact ledit échantillon avec un agent déclenchant la génération de ladite enzyme protéolytique dans l'échantillon ;
b) générer une courbe de signal fluorescent dans le temps en mesurant, dans le temps pendant que ladite enzyme protéolytique est générée dans l'échantillon, l'évolution dans le temps d'au moins un signal de fluorescence expérimental $F_{exp}.t$ émis par l'échantillon en résultat de la libération d'un groupe fluorescent du substrat fluorogène spécifique à ladite enzyme protéolytique lors de l'action enzymatique de l'enzyme protéolytique générée sur ledit substrat fluorogène ;
c) calculer $F_{max}$ à partir de $F_0$ obtenue expérimentalement et la constante $M = F_{max}/F_0$ pouvant être obtenue ou étant obtenue à partir d'une expérience de référence ;
d) générer une courbe de rapport normalisé dans le temps en convertissant les valeurs de $F_{exp.t}$ mesurées à l'étape b) en valeurs définies comme les valeurs de rapport normalisé $R_t$ selon l'équation $R_t = (F_{exp.t}-F_0)/(F_{max}-F_0)$, en utilisant $F_0$ et $F_{max}$ obtenues aux étapes a, b et c ;
e) générer une courbe de génération d'enzyme protéolytique dans le temps en appliquant la formule $E_t = dR_t/dt.(S_0+K_m/(1-R_t))/(k_{cat})$, où $S_0$ est la concentration initiale de substrat et $K_m$ et $k_{cat}$ sont des constantes cinétiques qui régissent l'interaction entre l'enzyme protéolytique et le substrat fluorogène.

3. Procédé selon la revendication 1 ou 2, dans lequel $F_0$ est la valeur de fluorescence pendant le temps de latence de génération d'enzyme et/ou dans lequel l'enzyme protéolytique est une thrombine et/ou plasmine.

4. Procédé selon la revendication 1, 2 ou 3, dans lequel le substrat fluorogène comprend en outre une quantité fixe de produit fluorescent qui est comprise entre 0,1 % et 10 % de la concentration de substrat fluorogène dans l'échantillon.

5. Procédé selon l'une quelconque des revendications 1 à 4, dans lequel le substrat fluorogène est un substrat fluorogène à base de rhodamine, de préférence un peptide à base de rhodamine, englobant de préférence un fluorophore Rhodamine 110, ou est un substrat fluorogène à base d'AMC, dans lequel, facultativement, l'échantillon est complété par une quantité du produit fluorescent correspondant aux substrats fluorogènes ou d'un produit fluorescent différent, égale entre 0,1 % et 10 % du substrat fluorogène dans l'échantillon ; et/ou, dans lequel le substrat fluorogène comprend au moins un oligopeptide ayant une séquence de 1 à 30 résidus d'acide aminé couplés à une molécule fluorescente, dans lequel l'oligopeptide a de préférence une lysine terminale ou une arginine terminale couplée à la molécule fluorescente.

6. Procédé selon la revendication 5, dans lequel le substrat fluorogène est un monopeptide à base de Rhodamine 110, dans lequel l'un des groupes NH réactifs symétriques dans la Rhodamine 110 est lié à l'oligopeptide au niveau d'un résidu terminal lysine ou arginine et l'autre groupe NH réactif est acétylé ; et/ou, dans lequel l'oligopeptide dans le substrat est couplé chimiquement à un groupe hydrophile ayant un poids moléculaire d'au moins 5 kD, tel qu'un groupe polyéthylèneglycol (PEG) ; et/ou, dans lequel l'oligopeptide du substrat a la séquence d'acides aminés : Gly-Gly-Arg, Gly-Pro-Arg, Ile-Pro-Arg, Phe-acide Pipécolique (PIP)-Arg, Val-Leu-Lys, Gly-Pro -Lys ou Ala-Phe-Lys.

7. Procédé selon l'une quelconque des revendications 1 à 6, dans lequel l'enzyme protéolytique est une thrombine et/ou une plasmine et dans lequel l'agent déclenchant la génération de thrombine et/ou de plasmine est choisi parmi le groupe constitué de : ions calcium, phospholipides, facteur tissulaire (TF), facteur tissulaire soluble (sTF), thromboplastine, kaolin, acide ellagique, activateur du plasminogène, en particulier activateur tissulaire du plasminogène (tPA), urokinase et streptokinase ; et/ou, dans lequel l'échantillon biologique est un échantillon de sang citraté, de préférence un échantillon de plasma citraté et/ou sans plaquette, un échantillon de plasma pauvre en plaquettes (PPP) ou un échantillon de plasma riche en plaquettes (PRP) ; et/ou, dans lequel, pour l'essai, l'échantillon biologique est déposé dans un conteneur ou sur un support, tel qu'une puce, de préférence une puce microfluidique, ledit conteneur ou support ayant le substrat fluorogène adsorbé sur une matrice opaque solide placée à l'intérieur du conteneur ou adsorbé sur le support.

8. Procédé selon l'une quelconque des revendications 1 à 7, dans lequel les courbes de génération de thrombine et de plasmine dans le temps sont générées simultanément, en utilisant un substrat fluorogène qui est sensible aux deux enzymes ou en utilisant un mélange d'un substrat fluorogène de plasmine et d'un substrat fluorogène de thrombine qui génèrent le même produit fluorescent.

9. Procédé de détection ou de surveillance *in vitro* d'une maladie hémostatique ou d'une maladie thrombotique, ou de surveillance du traitement d'une maladie hémostatique ou d'une maladie thrombotique, en mesurant l'effet sur la génération de thrombine et/ou de plasmine de l'administration de médicaments ou agents hémostatiques, antithrombotiques ou pro- ou anti-thrombolytiques, en mettant en œuvre un procédé selon l'une quelconque des revendications 1 à 8.

10. Procédé de détection ou de surveillance *in vitro* de l'interaction d'une substance candidate sur une activité thrombine et/ou plasmine dans un échantillon biologique, en particulier un échantillon de sang total, en mettant en œuvre un procédé selon l'une quelconque des revendications 1 à 9, dans lequel ladite substance candidate est ajoutée à l'échantillon à analyser ou est ajoutée à l'échantillon pendant la génération de thrombine et/ou de plasmine, de préférence pour surveiller une interaction de facteurs ou médicaments de coagulation ou de facteurs ou médicaments anticoagulants, de manière plus préférée d'inhibiteurs de thrombine et/ou de plasmine ou d'activateurs de plasmine, de manière plus préférée pour cribler des substances afin de déterminer leur capacité d'interaction avec la génération de thrombine et/ou de plasmine.

**11.** Procédé selon l'une quelconque des revendications 1 à 10 pour une mesure *in vitro* d'une capacité de génération de thrombine ou de plasmine dans l'échantillon biologique en produisant la courbe de génération de thrombine ou de plasmine et en évaluant l'aire sous la courbe (Potentiel de Thrombine Endogène (ETP) ou Potentiel de Plasmine Endogène), la valeur maximale de la courbe (pic) et/ou la mesure du temps jusqu'au pic de thrombine et/ou de plasmine.

**12.** Procédé de détermination *in vitro* de l'activité dans le temps de plasmine dans un échantillon biologique obtenu auprès d'un individu, comprenant les étapes consistant à :

i) effectuer le procédé selon l'une quelconque des revendications 1 à 11 de détermination *in vitro* de l'activité dans le temps de thrombine dans ledit échantillon biologique, dans lequel seule une génération de thrombine est déclenchée, et
ii) effectuer le procédé selon l'une quelconque des revendications 1 à 11 de détermination *in vitro* de l'activité dans le temps de thrombine et de plasmine dans l'échantillon biologique analysé à l'étape i), dans lequel une génération de thrombine et une génération de plasmine sont toutes deux déclenchées, et
iii) soustraire la sortie obtenue en i) à partir de la sortie obtenue en ii) pour obtenir une représentation de l'activité dans le temps de la plasmine pour l'échantillon analysé.

## Fig. 1

## Fig. 2

## Fig. 3

## Fig. 4

## Fig. 5A

## Fig. 5B

*Fig. 5C*

*Fig. 5D*

## Fig. 5E

## Fig. 5F

## Fig. 6A

## Fig. 6B

## Fig. 7A

## Fig. 7B

*Fig. 7C*

*Fig. 8A*

## Fig. 8B

## Fig. 8C

## Fig. 8D

## Fig. 9A

## Fig. 9B

Fig. 9C

Fig. 9D

## Fig. 10A

## Fig. 10B

*Fig. 10C*

*Fig. 11A*

*Fig. 11B*

*Fig. 11C*

## Fig. 11D

## Fig. 12A

*Fig. 12B*

*Fig. 12C*

# EP 3 363 912 B1

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- WO 03093831 A
- WO 2009098313 A
- WO 2006117246 A
- WO 2007141023 A
- WO 2006072602 A
- FR 2978163
- FR 2978164
- WO 0052199 A
- WO 2003093831 A1
- FR 2978163 P

### Non-patent literature cited in the description

- **HEMKER HC ; WIELDERS S ; KESSELS H ; BÉGUIN S.** Continuous registration of thrombin generation in plasma, its use for the determination of the thrombin potential. *Thromb.Haemost,* 1993, vol. 70, 617-624
- **HEMKER HC ; GIESEN PL ; RAMJEE M ; WAGENVOORD R ; BÉGUIN S.** The thrombogram, monitoring thrombin generation in platelet-rich plasma. *Thromb.Haemost,* 2000, vol. 83, 589-591
- **HEMKER et al.** *Thromb Haemost,* 2000, vol. 83, 589-91
- **HEMKER et al.** *Pathophysiol Haemost Thromb,* 2002, vol. 32, 249-253
- **HEMKER et al.** *Pathophysiol Haemost Thromb,* 2003, vol. 33, 4-15
- **WAGENVOORD RJ ; DEINUM J ; ELG M ; HEMKER HC.** The paradoxical stimulation by a reversible thrombin inhibitor of thrombin generation in plasma measured with thrombinography is caused by alpha-macroglobulin-thrombin. *Journal of thrombosis and haemostasis : JTH,* 2010, vol. 8, 1281-9
- **NINIVAGGI M ; APITZ-CASTRO R ; DARGAUD Y ; DE LAAT B ; HEMKER HC ; LINDHOUT T.** Whole blood thrombin generation monitored with a calibrated automated thrombogram-based assay. *Clin.Chem.,* 2012, vol. 58, 1252-1259
- **HEMKER et al.** Continuous registration of thrombin generation in plasma, its use for the determination of the thrombin potential. *Thromb.Haemost,* 1993, vol. 70, 617-624
- **WAGENVOORD et al.** The paradoxical stimulation by a reversible thrombin inhibitor of thrombin generation in plasma measured with thrombinography is caused by alpha-macroglobulin-thrombin. *Journal of thrombosis and haemostasis,* 2010, vol. 8, 1281-9
- **NINIVAGGI et al.** Whole blood thrombin generaton monitored with a calibrated automated thrombogram-based assay. *Clin.Chem.,* 2012, vol. 58, 1252-1259